(19) Europäisches Patentamt
European Patent Office
Office européen des brevets

(11) EP 1 561 449 B1

(12) EUROPÄISCHE PATENTSCHRIFT

(45) Veröffentlichungstag und Bekanntmachung des Hinweises auf die Patenterteilung:
**05.08.2009 Patentblatt 2009/32**

(51) Int Cl.:
***A61K 6/093*** *(2006.01)* ***A61K 6/10*** *(2006.01)*
***C08G 77/12*** *(2006.01)* ***C08G 77/20*** *(2006.01)*
***C08L 83/04*** *(2006.01)*

(21) Anmeldenummer: **04025383.3**

(22) Anmeldetag: **26.10.2004**

(54) **Über Hydrosilylierungs-Reaktion additionsvernetzende Zweikomponenten-Dentalmaterial mit starren und/oder voluminösen Gruppen sowie mit hoher Biegefestigkeit und E-Modul**

Addition cross-linking two-component silicon material containing rigid and/or bulky groups annd having a high flexural strength and E-modul

Matériau de type silicone à deux composants, à réticulation par addition, comprenant des groupes rigides et/ou volumineux et présentant une haute résistance à la flexion et un module recouvert

(84) Benannte Vertragsstaaten:
**CH DE ES FR GB IT LI**

(30) Priorität: **03.02.2004 DE 102004005562**

(43) Veröffentlichungstag der Anmeldung:
**10.08.2005 Patentblatt 2005/32**

(73) Patentinhaber: **Kettenbach GmbH & CO. KG**
**35713 Eschenburg (DE)**

(72) Erfinder:
- **Bublewitz, Alexander, Dr.**
**35745 Herborn (DE)**
- **Reber, Jens-Peter**
**58540 Meinerzhagen (DE)**
- **Nagel, Ulrich, Prof. Dr.**
**72076 Tübingen (DE)**

(74) Vertreter: **KEIL & SCHAAFHAUSEN**
**Patentanwälte**
**Cronstettenstraße 66**
**60322 Frankfurt am Main (DE)**

(56) Entgegenhaltungen:
**EP-A- 0 614 655** **EP-B- 0 894 117**
**WO-A-20/04052994** **US-A- 5 086 148**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 561 449 B1

## Beschreibung

Die vorliegende Erfindung betrifft ein über Hydrosilylierung additionsvernetzendes Zweikomponenten-Dentalmaterial enthaltend eine oder mehrere Verbindungen mit Vinylgruppen im Molekül, wenigstens eine Organohydrogensiliziumverbindung und wenigstens einen Katalysator. Außerdem betrifft die vorliegende Erfindung die Verwendung dieser additionsvernetzenden Zweikomponenten-Dentalmaterialien.

Bei verschiedenen Maßnahmen im Bereich der Dentalmedizin und Dentaltechnik, beispielsweise zur Zahnabdrucknahme, Bissregistrierung, Fixierung, Positionierung, Verschlüsselung, Wiederherstellung, Übertragung von Brakkets in der Kieferorthopädie und Remontage von Füllungen zum Einschleifen der Occlussion auf dem Metall, werden Materialien benötigt, welche von einem viskosen Zustand in einen besonders harten Zustand übergehen. Herkömmlicherweise werden hierzu additionsvernetzende Zweikomponenten-Siliconmaterialien eingesetzt. Während eine der beiden Komponenten dieser Zusammensetzungen vernetzungsfähige Polysiloxane, insbesondere Organopolysiloxane mit wenigstens zwei Vinylgruppen pro Molekül, sowie einen für die Vernetzungsreaktion notwendigen Hydrosilylierungskatalysator, typischerweise eine Platinverbindung, enthält, umfasst die andere Komponente wenigstens ein Hydrogenpolysiloxan, üblicherweise ein Organohydrogenpolysiloxan, als Vernetzer. Zusätzlich dazu können eine oder beide Komponenten verstärkende Füllstoffe, nicht-verstärkende Füllstoffe und/oder weitere Zusatz- und Hilfsmittel, wie Farbstoffe und dergl., enthalten. Zur Einstellung der die Verarbeitungszeit bestimmenden Aushärtungszeit werden den Siliconmaterialien Inhibitoren, bspw. kurzkettige vinylgruppenhaltige Organopolysiloxane, insbesondere Organodisiloxane, Benzotriazol oder Ethinylcyclohexanol, zugesetzt. Die mechanischen Eigenschaften, wie Härte und Elastizitätsmodul, werden insbesondere durch die Kettenlänge der eingesetzten Organopolysiloxane sowie die Art und die Menge an Füllstoffen bestimmt.

Aus der EP 0 522 341 A1 sind additionsvernetzende Massen auf Polysiloxanbasis bekannt, welche neben Organopolysiloxanen mit zwei oder mehreren Vinylgruppen im Molekül und einer Viskosität zwischen 100 und 200.000 mPa·s, Organohydrogenpolysiloxanen als Vernetzer, einem Katalysator und Farbstoffen, Compounds von hochdispersen aktiven Füllstoffen in Silikonöl und kurzkettige Organopolysiloxane mit zwei oder mehreren Vinylgruppen im Molekül enthalten. Die kurzkettigen Organopolysiloxane entsprechen der allgemeinen Formel

$$CH_2=CH-R_2SiO-(SiR_2O)_n-SiR_2-CH=CH_2$$

worin R gleiche oder verschiedene, von aliphatischen Mehrfachbindungen freie, einwertige, gegebenenfalls substituierte Kohlenwasserstoffe und n eine ganze Zahl zwischen 10 und 20 bedeutet. Allerdings weisen die in der EP 0 522 341 A1 offenbarten Siliconmaterialien lediglich eine Shore A-Härte von maximal 78, entsprechend einer Shore D-Härte von maximal 19, und ein Elastizitätsmodul im Zugversuch von maximal 9 MPa auf. Diese mechanischen Eigenschaften sind für die meisten dentalmedizinischen und dentaltechnischen Anwendungen, insbesondere für die Bissregistrierung, unzureichend.

In der EP 0 894 117 B1 werden additionsvernetzende Zweikomponenten-Siliconmaterialien offenbart, welche Organopolysiloxane mit zwei Vinylgruppen im Molekül, Organohydrogenpolysiloxane mit zwei oder mehr SiH-Gruppen und einem SiH-Gehalt von 1 bis 15 mmol/g als Vernetzer, einen Katalysator, verstärkende Füllstoffe sowie nicht-verstärkende Füllstoffe enthalten, wobei das Organopolysiloxan mit zwei Vinylgruppen im Molekül eine Viskosität zwischen 21 und 99 mPa·s, entsprechend einer Kettenlänge von 21 bis 69, aufweist. Diese ausgehärteten Massen weisen eine Shore D-Härte von wenigstens 35 und ein Elastizitätsmodul von größer 20 MPa (gemessen nach DIN 53457 oder 53455) auf, welche für die meisten dentalmedizinischen und dentaltechnischen Anwendungen schon ganz gut sind. Dennoch sind für diese Indikationen Materialien mit höheren mechanischen Festigkeiten und geringeren Elastizitäten wünschenswert.

Aufgabe der vorliegenden Erfindung ist es daher, ein Dentalmaterial zur Verfügung zu stellen, welches sich im Vergleich zu den bekannten Massen durch eine höhere Shore D-Härte und/oder ein höheres Elastizitätsmodul auszeichnet und bezüglich seiner weiteren anwendungstechnischen Eigenschaften, insbesondere Schrumpfungsgrad, Verarbeitungszeit, diesen zumindest vergleichbar ist.

Erfindungsgemäß wird diese Aufgabe durch ein über Wydrosilylierung additionsvernetzendes Zweikomponenten-Dentalmaterial enthaltend

a) eine oder mehrere Verbindungen mit Vinylgruppen im Molekül,
b) wenigstens eine Organohydrogensiliziumverbindung und
c) wenigstens einen Katalysator,

gelöst, wobei die wenigstens eine Verbindung a) und/oder die wenigstens eine Verbindung b)
als erste Struktureinheit wenigstens zumindest eine voluminöse und/oder starre Gruppe ausgewählt aus der Gruppe der tertiären Alkyl-, quartären Alkyl-, Cycloalkyl-, Cycloalkenyl-, Aryl-, Aralkyl-, Alkylarylgruppen, halogensubstituierten tertiären Alkylgruppen, halogensubstituierten quartären Alkylgruppen, halogenierten Arylgruppen, halogenierten Aralkylgruppen, halogenierten Alkylarylgruppen, besonders bevorzugt aromatischen und nichtaromatischen Mono-, Bis-, Oligo-, Polycyclusgruppen, Bisphenol A-, Bisphenol B-, Bisphenol F-Gruppen, 1,1,1-Tris(4-hydroxyphenyl)alkan-Gruppen, Norboman-Gruppen, Adamantan-Gruppen und Pentaerythrit-Gruppen, umfasst, sowie als zweite Struktureinheit wenigstens zwei alkenylfunktionelle und oder wenigstens zwei, bevorzugt wenigstens drei wasserstofffunktionelle Silyleinheiten der allgemeinen Formeln 1 umfasst

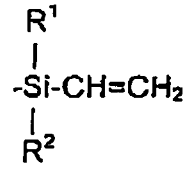 (Ia) oder 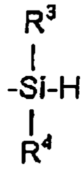 (Ib)

mit $R^1$, $R^2$ unabhängig voneinander ausgewählt aus der Gruppe, welche aus Alkylgruppen, Alkenylgruppen, Arylgruppen, Aralkylgruppen, Alkylarylgruppen, halogenierten Alkylgruppen, halogenierten Arylgruppen, halogenierten Aralkylgruppen, halogenierten Alkylarylgruppen, Cyanoalkylgruppen, Siloxygruppen, Cyloalkylgruppen und Cycloalkenylgruppen besteht, wobei Alkylgruppen besonders und Methylgruppen ganz besonders bevorzugt sind, und $R^3$. $R^4$ unabhängig voneinander H oder $R^1$, wobei Alkylgruppen besonders und Methylgruppen ganz besonders bevorzugt sind,
umfasst, wobei die zweite Struktureinheit

i) direkt,
ii) über ein Sauerstoffatom,
iii) über eine Spacergruppe gemäss Anspruch 1, oder
iv) über ein sauerstoffatom kombiniert mit einer Spacergruppe gemäß iii) an die erste Struktureinheit gebunden ist, und wobei die wenigstens eine Verbindung mit Vinylgruppen im Molekül a) einem sin-vinylgehalt von 2-10 mmol/g aufweist und die wenigstens eine organosilisium hydro-guverbindung b) einen sind gehalt von 4- 15 mmol/g aufweist.

Die vorliegende Erfindung basiert auf der überraschenden und für den Fachmann unerwarteten Erkenntnis, dass Zusammensetzungen enthaltend wenigstens eine Verbindung a) und/oder wenigstens eine Verbindung b) mit wenigstens einer voluminösen und/oder starren Gruppe neben den jeweils anderen Verbindungen a), b) bzw. c) zu Massen mit ausgezeichneten mechanischen Eigenschaften, insbesondere hervorragender Festigkeit und hohem Elastizitätsmodul, aushärten, welche zudem vorzüglich für dentalmedizinische und dentaltechnische Anwendungen geeignet sind. Insbesondere härten diese Materialien in einer für dentaltechnische und -medizinische Anwendungen ausreichend kurzen Zeit aus. Die hervorragenden mechanischen und anderen anwendungstechnischen Eigenschaften der erfindungsgemäßen Zusammensetzungen sind auf die voluminöse und/oder starre Gruppe der Verbindung a) bzw. auf die voluminöse und/oder starre Gruppe der Verbindung b) zurückzuführen. Die einzelnen Komponenten werden so ausgewählt, dass die Shore D-Härte der erfindungsgemäßen Dentalmaterialien im ausvulkanisierten Zustand (24 Stunden nach der Aushärtung bei Raumtemperatur) mehr als 69 und/oder die Biegefestigkeit mindestens 8 MPa, besonders bevorzugt mindestens 15 MPa und ganz besonders bevorzugt mindestens 19 MPa, und/oder das Elastizitätmodul im Biegeversuch (gemessen nach ISO 10477) mindestens 300 MPa, bevorzugt mindestens 600 MPa und ganz besonders bevorzugt mindestens 900 MPa, beträgt. Gleichzeitig beträgt die Verarbeitungszeit vorzugsweise weniger als 10 Minuten und besonders bevorzugt weniger als 5 Minuten.

Prinzipiell sind als erste Struktureinheit, d.h. als voluminöse und/oder starre Gruppe, der wenigstens einen Verbindung a) alle starren und/oder voluminösen Gruppen gemäss Anspruch 1 geeigriet, wobei unter starren Gruppen im Sinne der vorliegenden findung Gruppen mit definierter, unflexibler dreidimensionaler Struktur verstanden werden. Voluminöse Gruppe im Sinne der vorliegenden Erfindung bezeichnet solche Gruppen, welche aufgrund deren Größe, unter Berücksichtigung der gegenseitigen Orientierung der Vinylgruppen und oder der sind Gruppen im Molekul gewährleisten, dass der Abstand der jeweils wenigstens zwei Vinylgruppen pro Molekül der Verbindung a) voneinander wenigstens 0,3, insbesondere mindestens 0,45, wenigstens 0,5, mindestens 0,55, mindestens 0,6, wenigstens 0,7, mindestens 0,8 bzw. wenigstens 0,9 nm, beträgt. Daraus folgt, dass mit abnehmenden Winkel der Vinylgruppen untereinander im Molekül die erste Struktureinheit einen umso größeren Radius einer um die Gruppe gedachten Kugel aufweisen muss, um die notwendigen oben genannten Abstände der Vinylgruppen untereinander zu reichen. Die vorliegende Erfindung basiert auf der überraschenden Erkenntnis, dass bei einem Abstand der Vinylgruppen oberhalb der oben genannten Grenzwerte ein unerwünschter Chelateffekt infolge Annäherung zweier Vinylgruppen eines Moleküls an ein Platinatom zuverlässig vermieden werden kann. Die zuvor genannten Strukturparameter gelten für die Verbindungen b) entsprechend.

Erfindungsgemäß ist die erste Struktureinheit, d.h. die voluminöse und/oder starre Gruppe, eine solche, die aus der Gruppe, welche aus tertiären Alkyl-, quartären Alkyl-, Cycloalkyl-, Cycloalkenyl-, Aryl-, Aralkyl-, Alkylarylgruppen, halogensubstituierten tertiären Alkylgruppen, halogensubstituierten quartären Alkylgruppen, halogenierten Arylgruppen, halogenierten Aralkylgruppen, halogenierten Alkylarylgruppen, besonders bevorzugt aromatischen und nichtaromatischen Mono-, Bis-, Oligo-, Polycyclusgruppen, Bisphenol A-, Bisphenol B-, Bisphenol F-Gruppen, 1,1,1-Tris(4-hydroxyphenyl)alkan-Gruppen, Norbornan-Gruppen, Adamantan-Gruppen und Pentaerythrit-Gruppen, besteht, ausgewählt ist.

Als erste Struktureinheit besonders geeignet erwiesen haben sich insbesondere folgende Strukturen erwiesen:

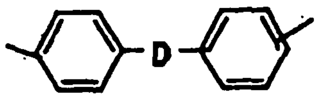 , 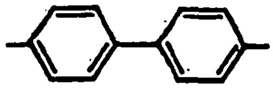 , 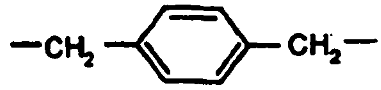

o, m, p 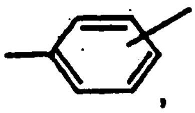 , 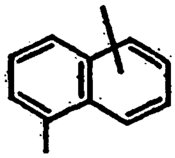 , 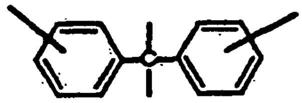 , 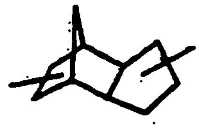

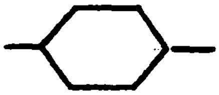 , 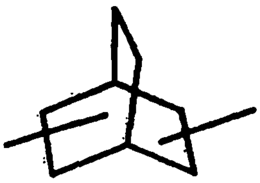 , 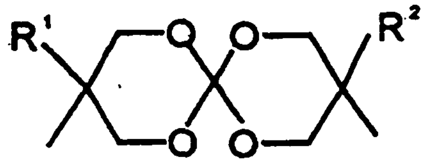

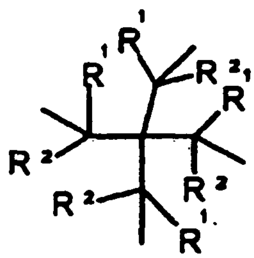 , 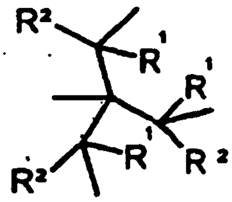 , 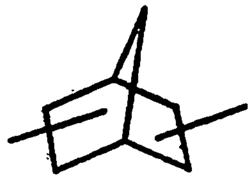

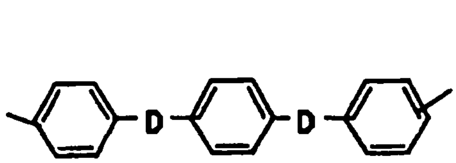 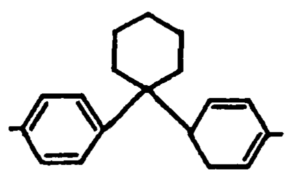

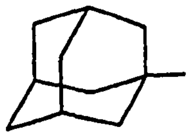
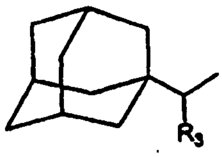
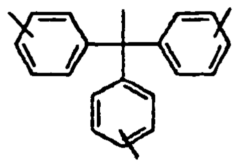

worin $R^1$, $R^2$, $R^3$ wie in den Formeln I definiert oder Wasserstoff sind, D= $CR^3R^4$ mit $R^3$ und $R^4$ wie oben definiert, insbesondere D gleich $CH_2$, $C(CH_3)_2$, CMePh mit Me=$CH_3$ und Ph=$C_6H_5$ oder $CPh_2$.

Ganz besonders bevorzugt als erste Struktureinheit sind Bisphenol A

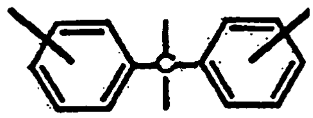

Bisphenol A-Derivate und Trisphenolverbindungen

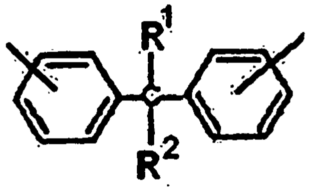
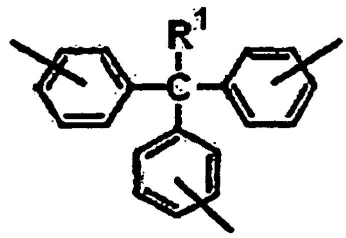

mit $R^1$, $R^2$ wie oben definiert.

Als zweite Struktureinheit, d.h. als wasserstoff- und/oder alkenylfunktionelle Silyleinheit, haben sich insbesondere solche der allgemeinen Formeln Ia oder Ib erwiesen, in denen die Reste $R^1$, $R^2$ bzw. $R^3$, $R^4$ unabhängig voneinander aus der Gruppe, welche aus Alkylgruppen, Alkenylgruppen, Arylgruppen, Aralkylgruppen, Alkylarylgruppen, halogenierten Alkylgruppen, halogenierten Arylgruppen, halogenierten Aralkylgruppen, halogenierten Alkylarylgruppen, Cyanoalkylgruppen, Siloxygruppen, Cyloalkylgruppen und Cycloalkenylgruppen besteht, ausgewählt sind, wobei $R^3$, $R^4$ auch Wasserstoffreste sein können. Besonders bevorzugt sind die Reste $R^1$, $R^2$ unabhängig voneinander Alkylgruppen, Arylgruppen, Aralkylgruppen oder Alkylarylgruppen, ganz besonders bevorzugt Methyl- oder Phenylgruppen, sowie die Reste $R^3$, $R^4$ unabhängig voneinander Wasserstoff, Alkylgruppen, Arylgruppen, Aralkylgruppen oder Alkylarylgruppen, ganz besonders bevorzugt Methyl- oder Phenylgruppen.

Gemäß einer ersten Ausführungsform der vorliegenden Erfindung enthält das additionsvernetzende Zweikomponenten-Dentalmaterial wenigstens eine Verbindung a), b) und c), wobei die wenigstens eine Verbindung a) und/oder die wenigstens eine Verbindung b) aus wenigstens einer der genannten ersten sowie wenigstens einer der genannten zweiten Struktureinheit besteht, wobei die wenigstens eine erste Struktureinheit direkt an die wenigstens eine zweite Struktureinheit gebunden ist und somit unter eine der allgemeinen Formeln II

$$H_2C=CH\text{-}SiR^1R^2\text{-}E_{n1}\text{-}SiR^1R^2\text{-}CH=CH^2 \qquad (IIa)$$

und/oder

$$H\text{-}SiR^3R^4\text{-}E_{n1}\text{-}SiR^3R^4\text{-}H \qquad (IIb),$$

wobei die Reste $R^1$, $R^2$, $R^3$, $R^4$ wie in Formel I definiert sind, E die erste Struktureinheit bezeichnet und $n_1$ eine ganze Zahl ≥ 1, vorzugsweise 1, ist,

fällt.

Vorzugsweise weist die unter die Formel IIa fallende Verbindung a) bzw. die unter die Formel IIb fallende Verbindung b) nur eine starre und/oder voluminöse Gruppe auf ($n_1$=1), flankiert von zwei Struktureinheiten der allgemeinen Formel Ia oder Ib, wobei jedoch die Gruppe E durchaus auch mit zwei bis sechs Gruppen der allgemeinen Formeln I substituiert sein kann.

Ebenfalls gute Ergebnisse werden nach einer zweiten Ausführungsform der vorliegenden Erfindung erzielt, wenn die wenigstens eine Verbindung a) und/oder die wenigstens eine Verbindung b) aus wenigstens einer der genannten ersten sowie wenigstens einer der genannten zweiten Struktureinheiten besteht, wobei die wenigstens eine erste Struktureinheit über ein Sauerstoffatom an die wenigstens eine zweite Struktureinheit gebunden ist und somit unter die allgemeinen Formeln III

$$H_2C=CH\text{-}SiR^1R^2\text{-}O\text{-}E_{n1}\text{-}O\text{-}SiR^1R^2\text{-}CH=CH_2 \qquad \text{(IIIa)},$$

und/oder

$$H\text{-}SiR^3R^4\text{-}O\text{-}E_{n1}\text{-}O\text{-}SiR^3R^4\text{-}H \qquad \text{(IIIb)}$$

wobei die Reste $R^1$, $R^2$, $R^3$, $R^4$ und E sowie $n_1$ wie oben definiert sind,

fällt. Im Unterschied zu den Verbindungen gemäß Formeln II, weisen diese Substanzen Si-O-C-Bindungen auf, die nach dem Stand der Technik als hydrolyseempfindlich und demnach aufgrund der Hydrolyse durch den Speichel im Patientenmund für Dentalmaterialien als ungeeignet gelten. Überraschenderweise konnte im Rahmen der vorliegenden Erfindung nunmehr gefunden werden, dass Si-O-C-Bindungen in Verbindungen der allgemeinen Formeln III keineswegs allgemein hydrolyseempfindlich sind, sondern vielmehr hydrolysestabil sein können. Hydrolysestabil sind diese Bindungen nach den Erkenntnissen der vorliegenden Erfindungen dann, wenn das Kohlenstoffatom der Si-O-C-Bindung mindestens mit einer Alkyl-, Aryl- oder Aralkylgruppe - welche weitere Substituenten aufweisen kann - substituiert ist. Ferner sind auch hydrolyseempfindliche Verbindungen a) mit Si-O-C-Bindungen, bei denen das Kohlenstoffatom nur mit Wasserstoff substituiert ist, einsetzbar, wenn diese zusammen mit nicht Si-O-C-haltigen Organohydrogensiliziumverbindungen b) gemischt und ausgehärtet werden sowie umgekehrt hydrolyseempfindliche Verbindungen b) mit Si-O-C-Bindungen, bei denen das Kohlenstoffatom nur mit Wasserstoff substituiert ist, einsetzbar, wenn diese zusammen mit nicht Si-O-C-haltigen vinylgruppenhaltigen Verbindungen a) gemischt und ausgehärtet werden. Die erhaltenen Vulkanisate sind nach Wasserlagerung hydrolysestabil, was offenbar auf einen Abschirmungseffekt der hydrophoben Siliziumgruppen zurückzuführen ist.

Nach einer dritten Ausführungsform der vorliegenden Erfindung besteht die wenigstens eine Verbindung a) und/oder die wenigstens eine Verbindung b) des erfindungsgemäßen Dentalmaterials aus wenigstens einer der genannten ersten sowie wenigstens einer der genannten zweiten Struktureinheit, wobei die wenigstens eine erste Struktureinheit über einen Spacer A an die wenigstens eine zweite Struktureinheit gebunden ist und somit unter die allgemeinen Formeln IV

$$H_2C=CH\text{-}SiR^1R^2\text{-}A^1{}_{n2}\text{-}E_{n1}\text{-}A^2{}_{n3}\text{-}SiR^1R^2\text{-}CH=CH_2 \qquad \text{(IVa)}$$

und/oder

$$H\text{-}SiR^3R^4\text{-}A^1{}_{n2}\text{-}E_{n1}\text{-}A^2{}_{n3}\text{-}SiR^3R^4\text{-}H \qquad \text{(IVb)},$$

wobei die Reste $R^1$, $R^2$, $R^3$, $R^4$ und E sowie $n_1$ wie oben definiert sind, $n_2$, $n_3$ gleich oder verschieden und jeweils ganze Zahlen ≥ 1, vorzugsweise 1. sind sowie $A^1$, $A^2$ unabhängig voneinander ein Spacer gemäss Anspruch 1 ist, fällt.

Als Spacer geeignet haben sich im Rahmen der vorliegenden Erfindung die folgenden Gruppen erwiesen:

- $OCH_2CH_2$-, -$O(CH_2)_4$-, -O-$CR^1R^2$-$CR^1R^2$- sowie besonders bevorzugt -$CH_2CH_2CH_2(OCH_2CH_2)_4$-, -$CH_2$-$CH_2$-$CH_2$-, -O-$C(CH_3)_2$-$CH_2$-, -O-$CH(CH_3)$-$CH_2$-, -O-$CH(C_2H_5)$-$CH_2$-, -O-$CH(C_4H_9)$-$CH_2$- und -O-$CH(C_{10}H_{21})$-$CH_2$-.

Ebenfalls gute Ergebnisse werden nach einer vierten Ausführungsform der vorliegenden Erfindung erzielt, wenn die wenigstens eine Verbindung a) und/oder die wenigstens eine Verbindung b) aus wenigstens einer der genannten ersten sowie wenigstens einer der genannten zweiten Struktureinheit besteht, wobei die wenigstens eine zweite Struktureinheit über einen Spacer gemäß der dritten Ausführungsform, welcher über ein Sauerstoffatom an die wenigstens eine erste Struktureinheit gebunden ist, mit der ersten Struktureinheit verbunden ist.

Bevorzugt werden als Verbindung a) und/oder Verbindung b) solche mit nur einer voluminösen und/oder starren Gruppe, also solche In denen $n_1$ In den allgemeinen zuvor genannten Formeln II bis IV gleich 1 ist, eingesetzt

Die wenigstens eine Verbindung a) weist einen Si-Vinylgehalt von 2 bis 10 mmol/g auf.

Besonders gute anwendungstechnische Eigenschaften hinsichtlich Mechanik und optischen Eigenschaften zeigen die erfindungsgemäßen Dentalmaterialien, welche eine der folgenden Substanzen als Verbindung a) enthalten, wobei die Reste $R^1$, $R^2$, soweit nicht in den untenstehenden Formeln anders vermerkt, die in der Legende zu Formel la angegebenen Bedeutungen haben:

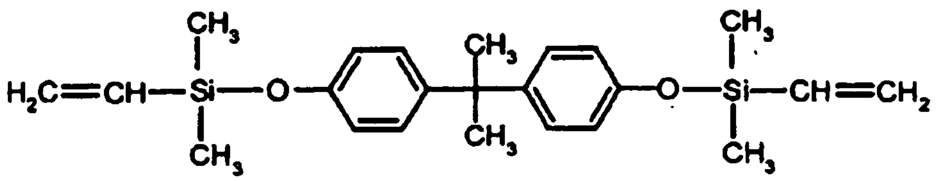

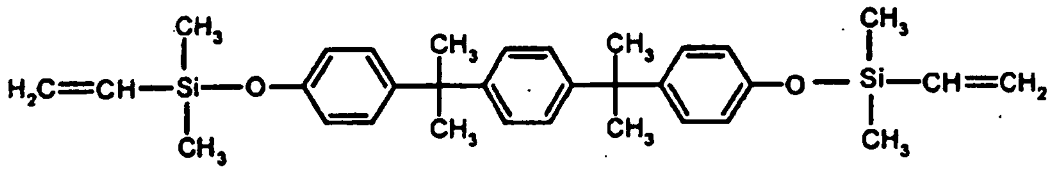

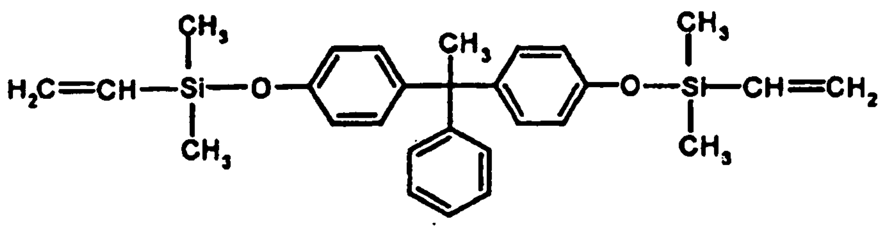

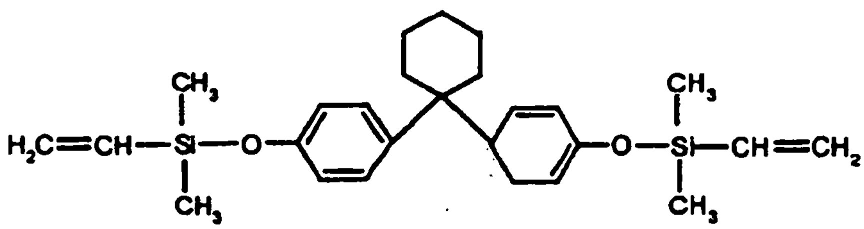

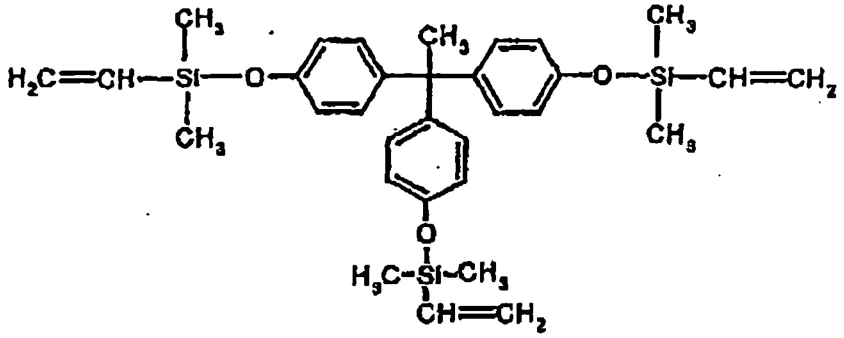

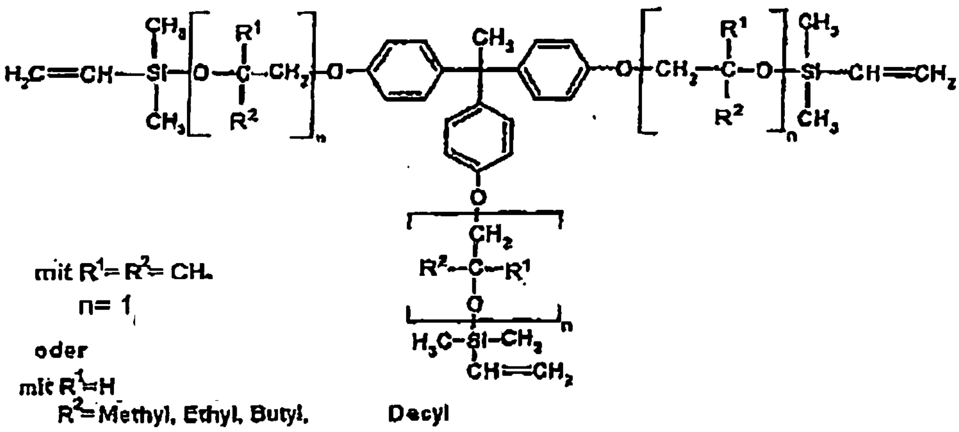

mit $R^1 = R^2 =$ CH.

$n = 1$

oder

mit $R^1 = H$

$R^2 =$ Methyl, Ethyl, Butyl, Decyl

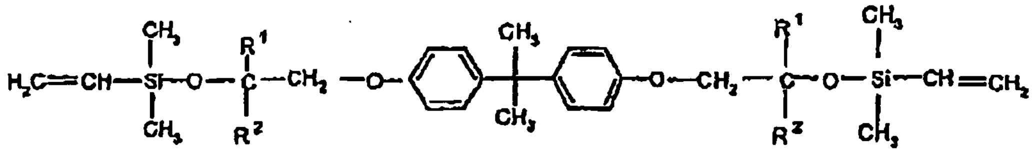

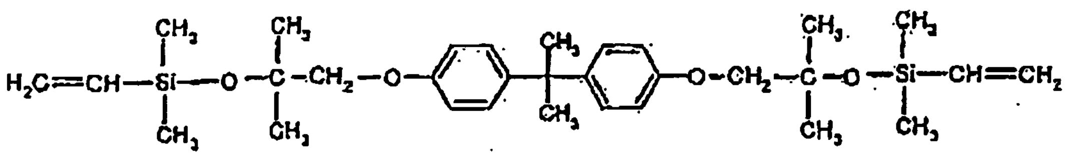

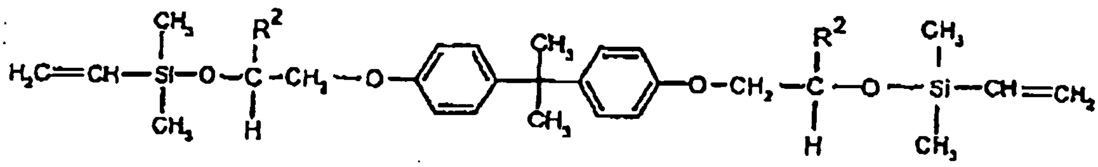

mit $R^2$= Methyl, Ethyl, Butyl Decyl

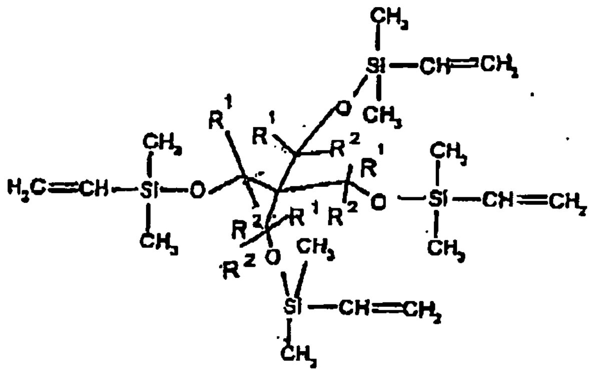

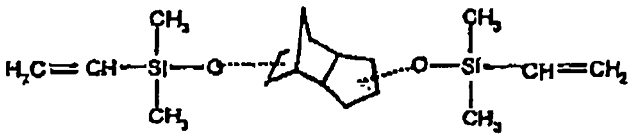

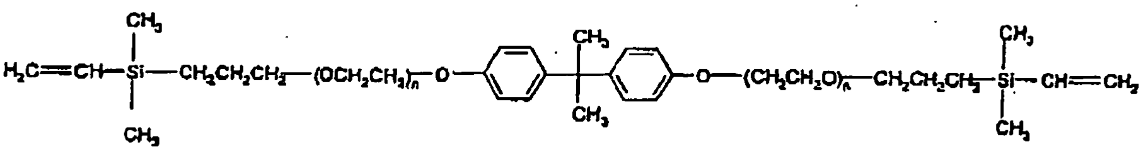

mit n= c oder 4

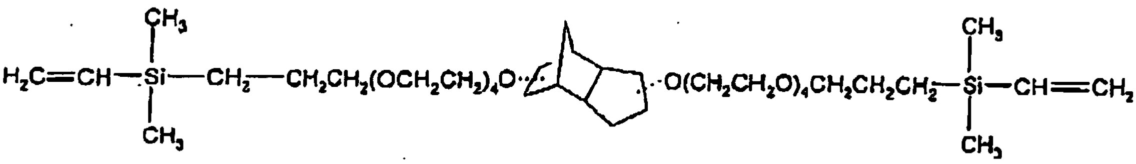

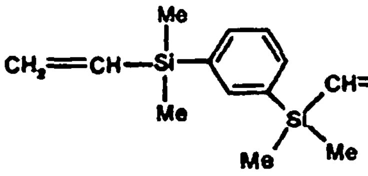
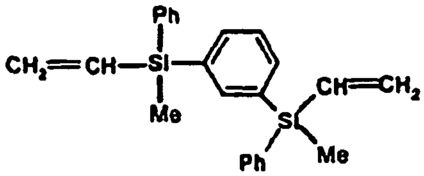

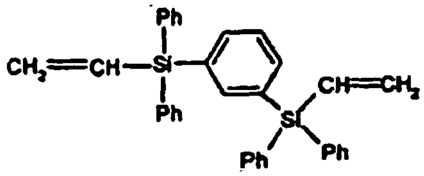
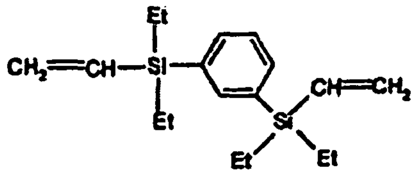

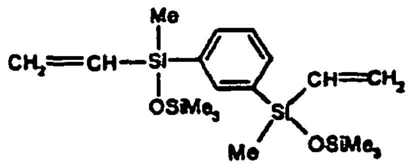
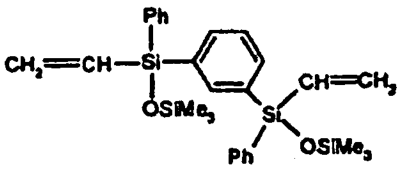

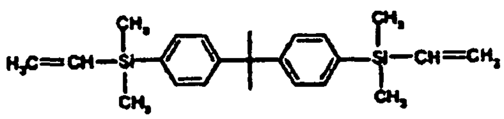
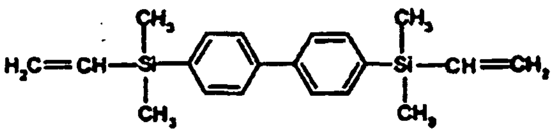

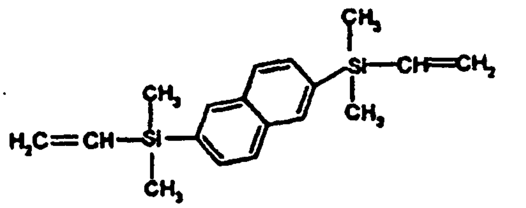

Erfindungsgemäß enthält das Dentalmaterial wenigstens eine die genannten zwei Struktureinheiten aufweisenden Verbindung a) oder wenigstens eine Verbindung b) und besonders bevorzugt jeweils mindestens eine entsprechende Verbindung a) und b). In dem Fall, dass nur eine die genannten zwei Struktureinheiten aufweisende Verbindung a) enthalten ist, können den erfindungsgemäßen Dentalmaterialien zusätzlich auch ein oder mehrere nach dem Stand der Technik bekannte Organopolysiloxane mit mindestens zwei Vinylgruppen pro Molekül und einer Viskosität (gemessen bei 20°C) zwischen 21 und 350.000 mPa·s (Komponenten $a_2$) zugesetzt sein, wobei der Gehalt an diesen Verbindungen vorzugsweise 0 bis 40 Gew.-%, besonders bevorzugt 0 bis 30 Gew.-% und ganz besonders bevorzugt 0 bis 20 Gew.-% beträgt.

Sofern das erfindungsgemäße Dentalmaterial nur wenigstens eine starre und/oder voluminöse Gruppe im Sinne der vorliegenden Erfindung aufweisende Verbindung a) aufweist, aber keine entsprechende Verbindung b), können als Vernetzer alle dem Fachmann für diesen Zweck bekannten Organohydrogenpolysiloxane, wobei sich insbesondere Polyalkyl-, Polyaryl-, Polyaralkyl-, Polyhalogenalkyl-, Polyhalogenaryl- und Polyhalogenaralkyl-Siloxane, welche im Molekül mindestens zwei, bevorzugt mindestens 3 an Siliciumatome gebundene Wasserstoffatome aufweisen, als geeignet erwiesen haben, eingesetzt werden. Sofern das erfindungsgemäße Dentalmaterial wenigstens eine starre und/oder voluminöse Gruppe im Sinne der vorliegenden Erfindung aufweisende Verbindung b) enthält, können diesem zusätzlich auch aus dem Stand der Technik bekannte Organohydrogenpolysiloxane (Komponente $b_2$), vorzugsweise solche mit wenigstens zwei Si-H-Gruppen pro Molekül und einem Si-H-Gehalt zwischen 0,1 und 15, besonders bevorzugt zwischen 4 und 14 und ganz besonders bevorzugt zwischen 5 und 13 mmol/g, sowie einer Viskosität (bei 20°C) von 5 bis 2.000

mPa·s, zugesetzt sein. In diesem Fall beträgt die Summe aus Organohydrogenpolysiloxane mit wenigstens eine starren und/oder voluminösen Gruppe im Sinne der vorliegenden Erfindung und Organohydrogenpolysiloxane gemäß Stand der Technik mindestens 1 Gewichtsprozent.

Als Organohydrogensiliziumverbindungen werden vorzugsweise

- lineare Organohydrogenpolysiloxane mit wenigstens zwei Si-H-Gruppen der allgemeinen Formel VI

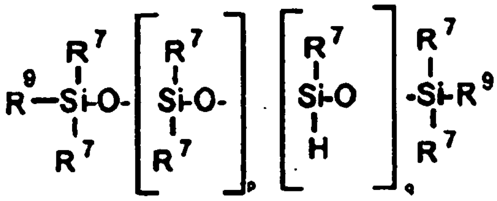

mit p = 0 bis 1500
mit q = 0 bis 1500, bevorzugt 2 bis 1500

mit $R^7$ = Alkyl- (z.B. Methyl-, Ethyl-, Isopropyl-), Aryl- (z.b. Phenyl-, Naphtyl-, Tolyl-, Xylyl-), Aralkyl- (Benzyl-, Phenylethyl-) und halogensubstitulerte Alkyl-und Aryl-Gruppen (z.B.3.3.3-Trifluorpropyl-, Chlorphenyl-, Difluorphanyl-), Cyanalkyl-, Cycloalkyl- und Cycloalkenyl-. Vorzugweise ist R = Methyl-.
mit $R^9$ = $R^7$ und/oder H

mit der Maßgabe, dass wenigstens 2 Si-Atome der Formel VI ein H-Atom tragen;

- Organohydrogenpolysiloxane mit T-Struktureinheiten der allgemeinen Formel VII

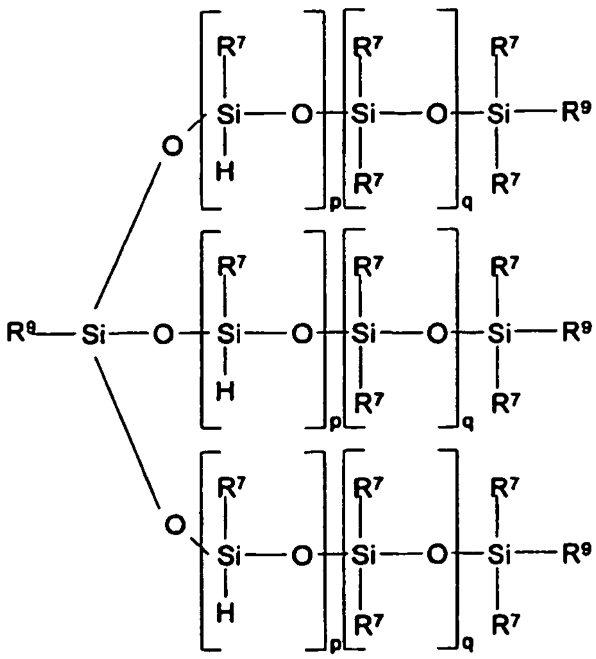

worin die Reste die in der Formel VI angegebene Bedeutung haben
und/oder
der Formel VIII:
$[SiO_{4/2}]\ [SiO_{½}R^1R^2H]_m$ mit m=0,9 bis 4, bevorzugt m=1 bis 4
und/oder

- Si-H-haltige QM-Harze bestehend aus $(R^9)_3SiO_{1/2}$, $(R^7)_2(M)SiO_{1/2}$ und $SiO_{4/2}$-Einheiten, wobei auch noch als T-Einheiten das trifunkctionelle $(R^9)_1SiO_{3/2}$ und als D-Einheiten das bifunktionelle $R^7R^9SiO_{2/2}$, wobei die Reste $R^7$ und $R^9$ die zuvor bezeichneten Bedeutungen haben, vorhanden sein können,

eingesetzt.

Besonders bevorzugt weist die wenigstens eine Organohydrogensilziumverblndung b) als erste Struktureinheit, d.h. als starre und/oder voluminöse Gruppe, aromatischen Mono-, Bis-, Oligo- oder Polycyclus gemäss Anspruch 1 und oder einen nichtaromatischen Mono-, Bis-, Oligo- oder Polycyclus gemäss Anspruch 1 auf sowie als wasserstofffunktionelle Silylgruppen solche, in denen die Reste $R^3$, $R^4$ unabhängig voneinander Wasserstoff, Alkyl-, Aryl-, Aralkyl- oder Alkylarylgruppen, ganz besonders bevorzugt Methyl- oder Phenylgruppen, sind, auf.

Beispiele für geeignete Organohydrogensiliziumverbindungen - wobei die Reste $R^1$, $R^2$, sofern in den nachfolgenden Formeln nicht anders vermerkt, die oben angegebenen Bedeutungen haben - sind:

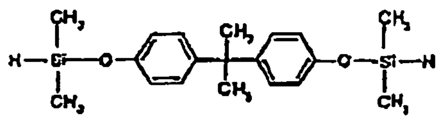

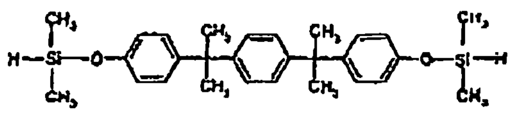

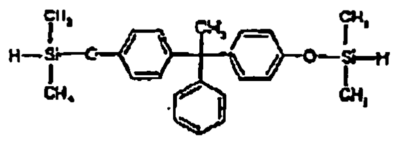

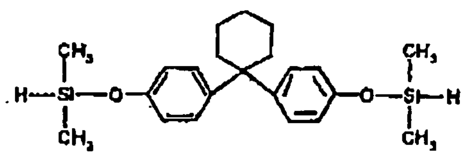

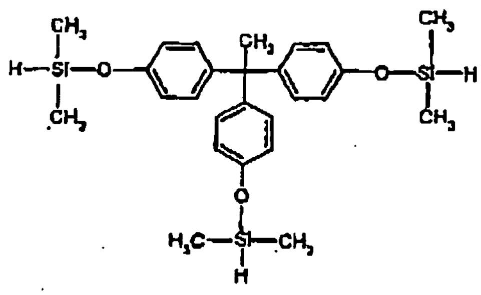

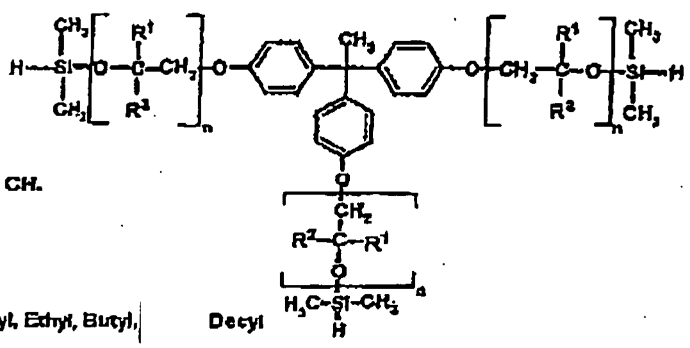

mit $R^1$ = $R^2$ = CH.
n = 1
oder
mit $R^1$ = H
$R^2$ = Methyl, Ethyl, Butyl, Decyl

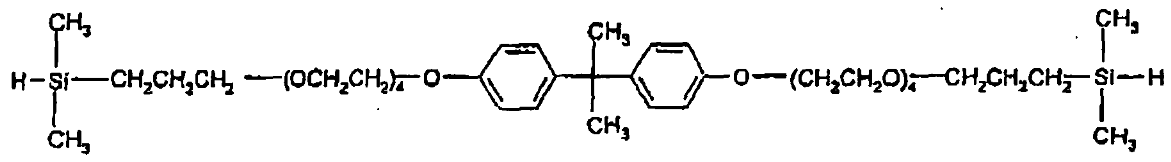

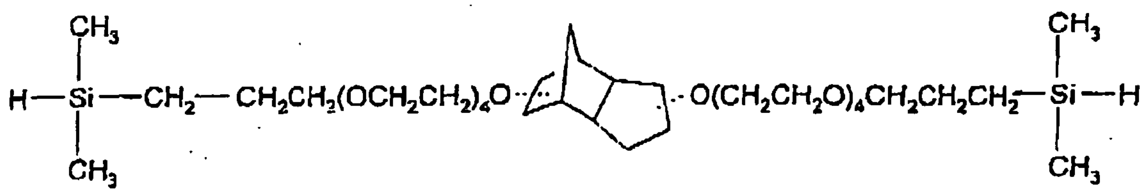

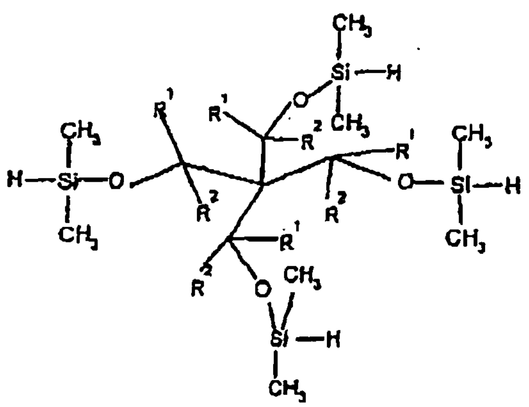

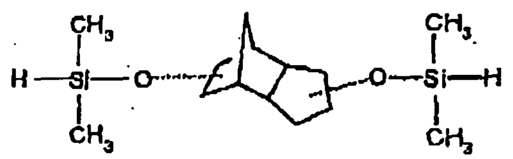

sowie Silopren® U430, U730, U830 und besonders bevorzugt U930 der Firma Bayer.

Um ein Ausgasen von Wasserstoffmolekülen aus den Vernetzermolekülen zu minimieren bzw. vollständig zu vermeiden, werden besonders bevorzugt Organohydrogensiliziumverbindungen eingesetzt, welche keine Si-H-Bindungen an jeweils benachbarten Siliziumatomen aufweisen, sondern solche, die bspw. folgende Struktureinheiten enthalten:

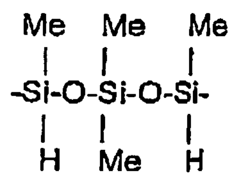

mit Me=$CH_3$

Ferner werden besonders bevorzugt Organohydrogensiliziumverbindungen mit einer T- oder Q-Struktur eingesetzt, da diese einen besonderes hohen Si-H-Gehalt aufweisen.

Die Organohydrogensiliziumverbindung b) weist einen SiH-Gehalt von 4 bis 15 mmol/g und ganz besonders bevorzugt von 7 bis 15 mmol/g sowie eine Viskosität von 1 bis 10.000 mPa·s, besonders bevorzugt von 5 bis 2.000 mPa·s (gemessen bei 20 °C mit einem Haake Viskosimeter) auf.

Als Katalysatoren (Komponente c) für die Hydrosilylierung können alle dem Fachmann zu diesem Zweck bekannte Substanzen, insbesondere Salze, Komplexe und kolloidal vorliegende Formen der Übergangsmetalle der 8. Nebengruppe, vorzugsweise der Metalle Platin, Palladium und Rhodium, eingesetzt werden. Bevorzugt enthalten die

erfindungsgemäßen Zusammensetzungen als Katalysator Platinkomplexe, welche bspw. aus Hexachloroplatinsäure oder aus entsprechenden Platinsalzen hergestellt werden, und besonders bevorzugt Platin-Divinyltetramethyldlsiloxan.

Neben wenigstens einer Verbindung a) und wenigstens einer Verbindung b), wobei wenigstens eine Verbindung a) und/oder wenigstens eine Verbindung b) eine starre und/oder voluminöse Gruppe im Sinne der vorliegenden Erfindung aufweist, sowie wenigstens einem Katalysator c) und ggf. einer oder mehrerer Verbindungen $a_2$), $b_2$) können die erfindungsgemäßen Zweikomponenten-Dentalmaterialien zusätzlich eine oder mehrere Substanzen jeweils aus einer oder mehreren der folgenden Gruppen enthalten:

d) verstärkende Füllstoffe,
e) nicht-verstärkende Füllstoffe,
f) Farbstoffe,
g) Feuchtigkeitsbinder,
h) Inhibitoren,
i) vinylgruppenhaltige, Si-H-gruppenhaltige und/oder SI-ORgruppenhaltige (mit R=H oder Alkyl) QM-Harze,
j) Compounds aus Organopolysiloxanen und verstärkenden Füllstoffen,
k) Tenside, Emulgatoren und/oder Stabilisatoren,
l) radioopake Substanzen,
m) $H_2$-absorbierende bzw. adsorbierende Substanzen und Substanzen, welche die $H_2$-Entwicklung eliminieren bzw. reduzieren, sowie
n) weitere Hilfs- und Zusatzstoffe, wie Rheologiehilfsmittel, Röntgenkontrastverbindungen, antimikrobielle Substanzen, adstringierende Substanzen oder dergl.

Als Komponente d) eignen sich insbesondere hochdisperse, aktive Füllstoffe mit einer BET-Oberfläche von mindestens 50 $m^2$/g (vgl. Schriftenreihe Pigmente Degussa Kieselsäuren, Nummer 12, Seite 5 sowie Nummer 13, Seite 3), wie Titandioxid, Aluminiumoxid, Zinkoxid, Zirkonoxid und besonders bevorzugt nassgefällte oder pyrogen gewonnene Kieselsäure. Die genannten Substanzen können hydrophil oder hydrophobiert vorliegen. Des weiteren können als verstärkende Füllstoffe Nanopartikel und faser- oder blättchenförmige Füllstoffe eingesetzt werden, wobei mineralische, faserförmige Füllstoffe, wie bspw. Wollastonit, und synthetische, faserförmige Füllstoffe, bspw. Glasfasern, Keramikfasern oder Kunststofffasern, bevorzugt sind. Nanopartikel im Rahmen der vorliegenden Erfindung bezeichnet speziell hergestellte anorganische oder organische Pulver, deren mittlere Korngröße weniger als 100 Nanometer beträgt.

Ferner können die erfindungsgemäßen Zusammensetzungen einen oder mehrere nicht-verstärkende Füllstoffe, bevorzugt solche mit einer BET-Oberfläche von weniger als 50 $m^2$/g, enthalten. Bevorzugt weisen die eingesetzten nichtverstärkenden Füllstoffe eine mittlere Korngröße von $\geq 0{,}1\ \mu m$ (UllmannEncyclopädie der Technischen Chemie, Band 21, Seite 523) auf. Besonders bevorzugt sind die nicht-verstärkende Füllstoffe ausgewählt aus der Gruppe, welche aus Metalloxiden, Metallhydroxiden, Metalloxidhydroxiden, Mischoxiden und Mischhydroxiden, besteht. Auch Füllstoffe wie Calciumcarbonat, Kieselgur, Diatomenerde, Talkum und Füllstoffe auf Kunststoffbasis, beispielsweise Polyalkyl (meth)acrylat, Polycarbonat, Polyvinylchlorid, Siliconharzpulver, Pulver auf Basis fluororganischer Verbindungen sowie organische und anorganische Hohlkugeln, Massivkugeln und Fasern können eingesetzt werden. Des weiteren können volle oder hohle Kunststoffteilchen, z. B. auch in sphärischer Form, auf deren Oberfläche anorganische Füllstoffpartikel eingebettet sind, verwendet werden. Bevorzugt sind Siliciumdioxid, insbesondere in Form von Quarz und seinen kristallinen Modifikationen, Quarzgut sowie, Quarzmehl, Cristobalit, Dentalgläser, Dentalkeramik, Aluminiumoxid, Calciumoxid und Aluminiumhydroxid.

Die unter d) und e) genannten Füllstoffe können auch oberflächenbehandelt (gecoated) vorliegen. Die Oberflächenbehandlung kann z. B. mit Silanen und Fettsäuren erfolgen, welche funktionelle Gruppen (z. B. Vinyl-, Si-Vinyl-, Allyl-, - SiH, Acryl- und Methacryl-) aufweisen können. Die Füllstoffe d) und e) können auf dem Fachmann bekannte Weise derart ausgewählt werden, dass opake oder transparente Dentalmaterialien erhalten werden.

Bei den unter f) genannten Farbstoffen handelt es sich um lösliche Farbstoffe oder um Pigmentfarbstoffe. Sofern die erfindungsgemäßen Dentalmaterialien für dentalmedizinische und dentaltechnische Anwendungen eingesetzt werden, enthalten diese als Farbstoffe vorzugsweise Lebensmittelfarbstoffe und/oder Eisenoxid-Dentalfarbstoffe. Des weiteren sind Farbpasten aus Polysiloxan- bzw. Mineralöl-Farbstoff-Formulierungen für diesen Zweck geeignet.

Als Feuchtigkeitsbinder g) können Zeolithe, wasserfreies Aluminiumsulfat, Molsieb, Kieselgur und Blaugel eingesetzt werden.

Als Inhibitoren h) können die erfindungsgemäßen Zweikomponenten-Dentalmaterialien u.a. alle Arten von Divinyldisiloxanen der allgemeinen Formel IX

$$CH_2{=}CH\text{-}SiR_2O\text{-}SiR_2\text{-}CH{=}CH_2$$

worin R gleiche oder verschiedene, ggf. substituierten Kohlenwasserstoffreste, wie Alkyl-, Alkenyl- und Alkinylgruppen,

bezeichnet,
enthalten.

Besonders bevorzugt werden als Inhibitoren Divinyltetraalkyldisiloxane und/oder Divinyltetramethyldisiloxan eingesetzt. Alternativ oder zusätzlich dazu können auch vinylgruppenhaltige cyclische Siloxane, bspw. Tetravinyltetramethylcyclotetrasiloxan, oder endständige Doppel- oder Dreifachbindungen enthaltende organische Hydroxylverbindungen, z.B. Ethinylcyclohexanol, als Inhibitoren eingesetzt werden.

Die unter i) genannten vinyl-, Si-H- und/oder Si-OR- (mit R=H oder Alkyl) gruppenhaltigen, festen oder flüssigen QM-Harze sind dadurch gekennzeichnet, dass sie als Q-Einheit das tetrafunktionelle $SiO_{4/2}$ und als M-Bausteine das monofunktionelle $R_3SiO_{1/2}$, wobei R = Vinyl-, Methyl-, Ethyl-, Phenyl- sein kann, enthalten. Darüber hinaus können auch noch als T-Einheiten das trifunktionelle $RSiO_{3/2}$ und als D-Einheiten das bifunktionelle $R_2SiO_{2/2}$ mit der gleichen Bedeutung für R wie oben genannt, vorhanden sein. Diese QM-Harze können in Organopolysiloxanen mit zwei oder mehr Vinylgruppen im Molekül und einer Viskosität von 21 bis 350.000 MPa·s gelöst vorliegen. Der Vinylgruppengehalt der genannten QM-Harze liegt vorzugsweise im Bereich von 0,1 bis 8 mmol/g, während der SiH-Gehalt vorzugsweise zwischen 0 und 15 mmol/g, besonders bevorzugt zwischen 0 und 1 mmol/g, und der Si-OR-Gehalt vorzugsweise zwischen 0 und 0,5 mmol/g liegt. Der niedrige SiOH-Gehalt der QM-Harze ist deshalb bevorzugt, weil dadurch ein Gasen durch Wasserstoffentwicklung vermieden wird. Auch der Anteil an flüchtigen Bestandteilen der QM-Harze sollte möglichst gering sein, damit die Dimensionsstabilität nicht beeinträchtigt wird.

Die gegebenenfalls eingesetzten Compounds j) sind bevorzugt aus Organopolysiloxanen mit zwei oder mehr Vinylgruppen im Molekül und einer Viskosität von 21 bis 350.000 MPa·s sowie den unter d) genannten verstärkenden Füllstoffen zusammengesetzt. Besonders bevorzugt werden unter Verwendung von Modifizierhilfsmitteln, z. B. Hexamethyldisilazan *in situ* hydrophobierte, Compounds eingesetzt.

Bei den ggf. als Tensid, Emulgator und/oder Stabilisator eingesetzten Komponenten k) kann es sich um anionische Tenside, insbesondere Alkylsulfate, Alkylbenzolsulfonate und -phosphate, kationische Tenside, insbesondere Tetraalkylammoniumhalogenide, nichtionische Tenside, insbesondere Alkyl- und Alkylphenyl-Polyalkylalkylenoxide, Fettsäurealkoxylate, Fettalkoholalkyloxylate und deren Alkylether und Alkylester, Fettsäurealkylolamide, Saccharosefettsäureester, Trialkylaminoxide, Silicontenside oder Fluortenside sowie amphotere Tenside, insbesondere sulfatierte oder oxyethylierte Kondensationsprodukte aus Alkylenphenolen und Formaldehyd, Ethylenoxid-Propylenoxid-Blockpolymerisate und/oder modifizierte Polysiloxane handeln. Ferner können die Tenside auch funktionelle Gruppen, wie z. B.-OH-, $-CH=CH_2$, $-OCO-(CH_3)C=CH_2$ und SiH enthalten. Darüber hinaus können selbstverständlich, wenn auch nicht bevorzugt, alle andere dem Fachmann zu diesem Zweck bekannte Verbindungen eingesetzt werden.

Als radioopake Substanzen I) können die erfindungsgemäßen Zweikomponenten-Dentalmaterialien bspw. barium-, strontium-, lanthan- oder zinkhaltige Gläser, Bariumsulfat, Zirkondioxid, Lanthanoxid oder keramische Füllstoffzusammensetzungen, welche Oxide von Lanthan, Hafnium und Seltenerdmetallen enthalten, aufweisen. Des weiteren können komplexe Schwermetallfluoride der allgemeinen Formel $M^{II}M^{IV}F_6$ oder $YF_3$ für diesen Zweck eingesetzt werden, wobei M" insbesondere ein Calcium-, Strontium- oder Bariumion und $M^{IV}$ insbesondere ein Titan-, Zirkon- oder Hafniumion ist. Ferner können als radioopake Substanzen am Siliconpolymer gebundene Atome bzw. Atomgruppen, die radioopake Eigenschaften besitzen, bspw. an Silicium gebundenes Jod, eingesetzt werden.

Bei den unter m) genannten $H_2$-Absorbern/Adsorbern handelt es sich vorzugsweise um feinverteiltes Palladium oder Platin bzw. deren Legierungen, die ggf. in Alumosilikaten enthalten sind. Weiterhin sind auch Substanzen einsetzbar, welche die $H_2$-Entwicklung eliminieren bzw. reduzieren, bspw. z. B. 3-Methyl-1-butin-3-ol und $CH_3Si[O-C(CH_3)_2-C=CH]_3$.

Der Gesamtgehalt an den Zusatz- und Hilfsstoffen f) bis h) und k) bis n) liegt bevorzugt zwischen 0 und 10, besonders bevorzugt zwischen 0 und 7 und ganz besonders bevorzugt zwischen 0 und 5 Gew.-%.

Vorzugsweise enthält das erfindungsgemäße DentaiMaterial. d. h. das Gemisch aus den beiden Komponenten des additionsvernetzenden 2-Komponenten-Systems, die nachstehend aufgeführten Substanzen in den jeweils angegebenen Mengenbereichen in Gew.-%, bezogen auf das gesamte Dentalmaterial:

$a_1$) 1 bis 90 Gew.-%, besonders bevorzugt 5 bis 75 Gew.-% und ganz besonders bevorzugt 10 bis 60 Gew.-% wenigstens einer Verbindung a) mit mindestens einer starren und/oder voluminösen Gruppe sowie wenigstens zwei vinylfunktionellen Silylgruppen im Molekül und einem Si-Vinylgehalt von 2 bis 10 mmol/g.

$a_2$) 0 bis 40 Gew.-%, besonders bevorzugt 0 bis 30 Gew.-% und ganz besonders bevorzugt 0 bis 20 Gew.-% wenigstens eines Organopolysiloxans mit mindestens zwei Vinylgruppen im Molekül und einem Si-Vinylgehalt von 0,5 bis 10 mmol/g, bevorzugt einem Si-Vinylgehalt von 1 bis 10 mmol/g und besonders bevorzugt einem Si-Vinylgehalt von 2 bis 10 mmol/g, sowie einer Viskosität von 21 bis 350.000 mPa·s,

$b_1$) 0 bis 90 Gew.-%, besonders bevorzugt 5 bis 75 Gew.-% und ganz besonders bevorzugt 10 bis 60 Gew.-% einer Organohydrogensitiziumverbindung-mit mindestens einer starren und/oder voluminösen Gruppe sowie wenigstens zwei silylgruppen mit mindestens 2, bevorzugt mindestens drei SiH-Gruppen und einem SiH-Gehalt von 4 bis 15

mmol/g und besonders bevorzugt von 7 bis 15 mmol/g,

$b_2$) 0 bis 50 Gew.-%, besonders bevorzugt 0 bis 40 Gew.-% und ganz besonders bevorzugt 0 bis 30 Gew.-% eines Organohydrogenpolysiloxans mit mindestens zwei, bevorzugt mindestens 3 SiH-Gruppen, und einem SiH-Gehalt von 0,1 bis 15 mmol/g, bevorzugt von 4 bis 15 mmol/g, besonders bevorzugt von 7 bis 15 mmol/g und ganz besonders bevorzugt von 7 bis 13 mmol/g, sowie einer Viskosität zwischen 5 und 2.000 mPa·s, wobei die Summe der Gewichtsprozente der Verbindungen $b_1$) und $b_2$) mindestens 1 Gew.-% beträgt,

c) 0,00001 bis 0,2 Gew.-%, vorzugsweise 0,0005 bis 0,1 Gew.-% wenigstens eines Katalysators zur Beschleunigung der Hydrosilylierungsreaktion, bezogen auf reines Metall,

d) 0 bis 50 Gew.-%, besonders bevorzugt 0,1 bis 40 Gew.-% und ganz besonders bevorzugt 0,5 bis 35 Gew.-% an verstärkenden Füllstoffen mit einer BET-Oberfläche von mindestens 50 $m^2$/g,

e) 0 bis 90 Gew.-%, besonders bevorzugt 0 bis 80 Gew.-% und ganz besonders bevorzugt 0 bis 75 Gew.-% an nicht verstärkenden Füllstoffen mit einer BET-Oberfläche von weniger als 50 $m^2$/g und einer mittleren Korngröße von wenigstens 0,1 μm,

f) 0 bis 5 Gew.-%, vorzugsweise 0 bis 2 Gew.-% wenigstens eines Farbstoffes,

g) 0 bis 30 Gew.-%, vorzugsweise 0 bis 5 Gew.-% an Feuchtigkeitsbinder,

h) 0 bis 1 Gew.-%, vorzugsweise 0 bis 0,6 und besonders bevorzugt 0 bis 0,1 Gew.-% an Inhibitoren,

i) 0 bis 40 Gew.-%, besonders bevorzugt 0 bis 30 Gew.-% und ganz besonders bevorzugt 0 bis 20 Gew.-% wenigstens eines vinylgruppenhaltigen und/oder SiH-haltigen und/oder SiOR-haltigen (mit R=H oder Alkyl) QM-Harzes mit einem SiH-Gehalt von 0 bis 15 mmol/g, besonders bevorzugt von 0 bis 1 mmol/g und/oder einem SiOR-Gehalt von 0 bis 0,5 mmol/g,

j) 0 bis 80 Gew.-%, vorzugsweise 0 bis 50 Gew.-% an Compounds aus vinylgruppenhaltigen Organopolysiloxanen und verstärkenden Füllstoffen,

k) 0 bis 10 Gew.-%, vorzugsweise 0 bis 5 Gew.-% an Tensiden, Emulgatoren und/oder Stabilisatoren,

l) 0 bis 90 Gew.-%, vorzugsweise 0 bis 80 Gew.-% an radioopaken Substanzen,

m) 0 bis 20 Gew.-%, vorzugsweise 0 bis 10 Gew.-% an $H_2$-Absorbern/Adsorbem oder an die $H_2$-Entwicklung reduzierenden bzw. eliminierenden Substanzen, sowie

n) 0 bis 20 Gew.-%, vorzugsweise 0 bis 15 Gew.-% an weiteren Hilfs- und Zusatzstoffen.

Die erfindungsgemäßen Zweikomponenten-Dentalmaterialien zeichnen sich im ausvulkanisierten Zustand durch eine hohe Shore D-Endhärte und/oder durch eine hohe Biegefestigkeit und/oder ein großes Elastizitätsmodul im Biegeversuch, bei gleichzeitig kurzer Abbindezeit aus. Diese Zusammensetzungen eignen sich daher für alle dentalmedizinischen und dentaltechnischen Anwendungen, in denen eine hohe Endhärte und eine geringe Elastizität des Materials gefordert ist. Insbesondere eignen sich die erfindungsgemäßen Materialien zur Bissregistrierung, zur Fixierung, zur Zahnabdrucknahme, zur Positionierung, als Zement, als provisorische Kronen, als Brückenmaterial, als provisorisches sowie als dauerhaftes Füllmaterial, zur Verschlüsselung, zur Wiederherstellung, zur Übertragung von Brackets in der Kieferorthopädie und zur Remontage von Füllungen zum Einschleifen der Occlussion auf dem Metall. In Verbindung mit Dentalgläsern (mittlerer Korndurchmesser von 10 bis 0,1 μm) und Nano-Füllstoffen (mittlerer Korndurchmesser kleiner 100 nm) lassen sich hervorragende mechanische Eigenschaften und optische Eigenschaften hinsichtlich Transluzenz erzielen, wie sie bspw. zur Erstellung von provisorischen Kronen und Brücken und für Füllungsmaterial gefordert werden.

Im folgenden wird die Erfindung anhand von den Erfindungsgedanken demonstrierenden, diesen jedoch nicht einschränkenden Beispielen erläutert.

**Synthesebeispiel 1**

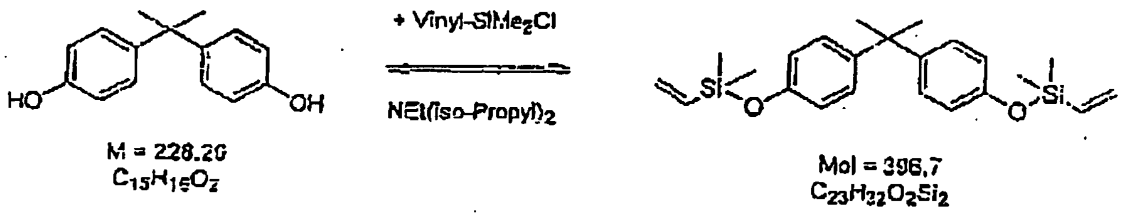


25 g (110 mmol) Bisphenol A wurden in 250 ml Triethylenglycoldimethylether/tert.-Butyltoluol (1:1) aufgelöst und anschließend dieser Lösung 75 ml NEt(iso-Propyl)$_2$ zugegeben. Beim Zutropfen von 35,8 ml (262 mmol) Vinyl-SiMe$_2$Cl bildete sich ein weißer Niederschlag. Nach 24 Stunden bei 90°C Ölbadtemperatur wurde der Niederschlag abfiltriert und die Lösungsmittel über ein U-Rohr unter Vakuum bei 120°C abgezogen.

Es wurden 42 g Produkt entsprechend einer Ausbeute von 96% erhalten.

**Synthesebeispiel 2**

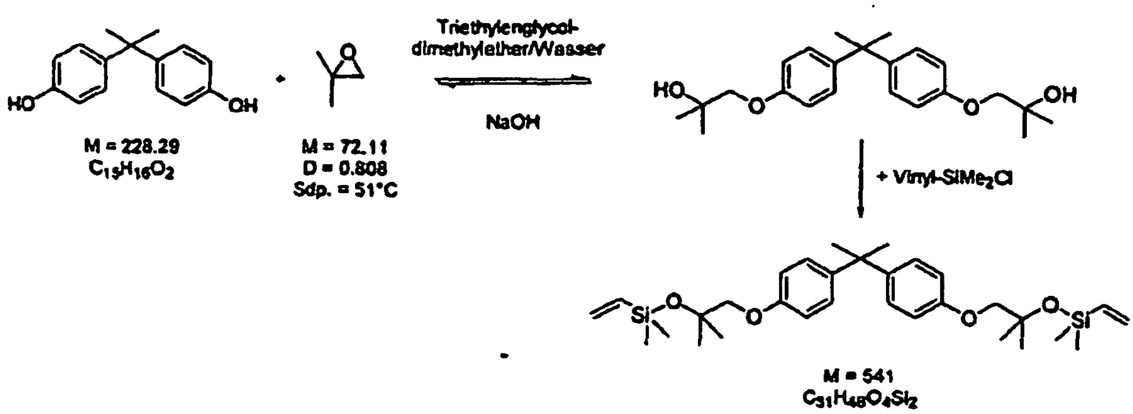


25 g (110 mmol) Bisphenol A wurden in 250 ml Triethylenglycoldimethylether gelöst und dazu eine konzentrierte NaOH-Lösung (11 mmol) zugegeben. Nach Zugabe von 100 ml Wasser und 39 ml 2,2-Dimethyloxiran wurde für 2 Tage bei 95 °C gerührt. Die Lösungsmittel wurden über ein U-Rohr im Vakuum bei 120 °C abgezogen. Anschließend wurde der Rückstand in Triethylenglycoldimethylether/tert.-Butyltoluol aufgelöst und 75 ml NEt(iso-Propyl)$_2$ zugegeben. Beim Zutropfen von 35,8 ml (262 mmol) Vinyl-SiMe$_2$Cl bildete sich ein weißer Niederschlag. Nach 24 Stunden bei 90°C Ölbadtemperatur wurde der Niederschlag abfiltriert und die Lösungsmittel über ein U-Rohr unter Vakuum bei 120°C abgezogen.

Es wurden 56 g einer braunen, zähen Flüssigkeit als Produkt entsprechend einer Ausbeute von 95% erhalten.

**Synthesebeispiel 3**

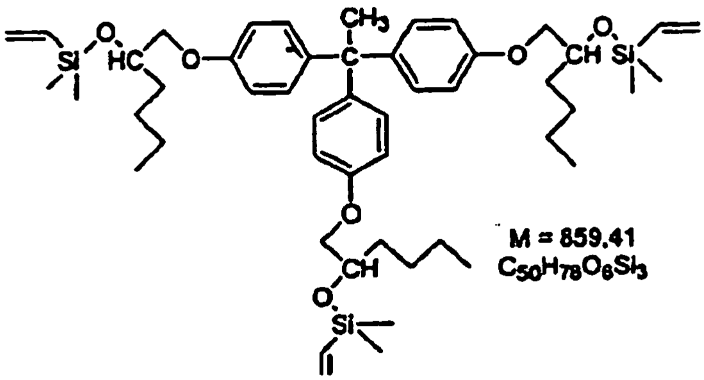

M = 859,41 $C_{50}H_{78}O_6Si_3$

50 g (163 mmol) 1,1,1-Tris-(4-hydroxyphenyl)-ethan wurden in 300 ml Tetraethylenglycoldimethylether gelöst und zu dieser Lösung eine konzentrierte NaOH-Lösung (25 mmol) zugegeben. Nach Zugabe von 100 ml Wasser und 88 ml (734 mmol) Butyloxiran wurde für 2 Tage bei 100°C gerührt. Die Lösungsmittel wurden über ein U-Rohr im Vakuum bei 140°C abgezogen. Es wurden 79 g (80%) einer zähen Flüssigkeit erhalten, die anschließend in 600 ml tert.-Butyltoluol und 168 ml NEt(iso-Propyl)$_2$ gelöst wurde.

Zu dieser Lösung wurden 80 ml (587 mmol) Vinyldimethylchlorsilan zugesetzt und 24 Stunden bei 90°C gerührt. Danach wurde die Mischung auf Raumtemperatur abgekühlt, der Niederschlag abfiltriert und das Lösungsmittel bei 120°C im Vakuum abgezogen.

Es wurden als Produkt 133 g einer zähen, noch fließfähigen, braunen Verbindung erhalten entsprechend einer Ausbeute von 95%.

**Synthesebeispiel 4**

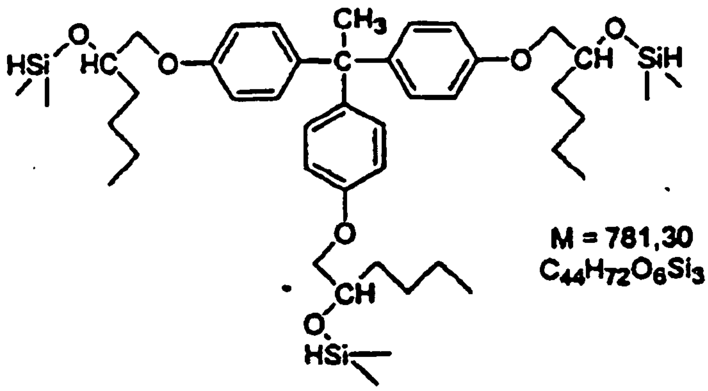

M = 781,30 $C_{44}H_{72}O_6Si_3$

50 g (163 mmol) 1,1,1-Tris-(4-hydroxyphenyl)-ethan wurden in 300 ml Tetraethylenglycoldimethylether gelöst und zu dieser Lösung eine konzentrierte NaOH-Lösung (25 mmol) zugegeben. Nach Zugabe von 100 ml Wasser und 88 ml (734 mmol) Butyloxiran wurde für 2 Tage bei 100°C unter Rückfluss gerührt. Die Lösungsmittel werden über ein U-Rohr im Vakuum bei 140°C abgezogen. Man erhielt 79 g (80%) einer zähen Flüssigkeit, die anschließend in 600 ml tert.-Butyltoluol und 168 ml NEt(iso-Propyl)$_2$ gelöst wurden.

Zu dieser Lösung wurden 64 ml (587 mmol) Dimethylchlorsilan zugesetzt und 24 Stunden bei 80°C gerührt. Danach wurde die Mischung auf Raumtemperatur abgekühlt, der Niederschlag abfiltriert und das Lösungsmittel bei 120°C im Vakuum abgezogen.

Es wurden als Produkt 114 g einer zähflüssigen, gelblichen Verbindung entsprechend einer Ausbeute von 90% erhalten.

**Synthesebeispiel 5**

**(Herstellung von 4,4'- Bis(vinyldimethylsilyl)biphenyl)**

1. Schritt: Synthese von 4,4'- Bis(ethoxydimethylsilyl)

In einem Glaskolben wurden 4,81 g (0,198 mol) getrocknetes Magnesium und 26,81 g (0,181 mol) $Me_2Si(OEt)_2$ vorgelegt und dieser Mischung 10 ml Tetrahydrofuran (THF) zugesetzt. Anschließend wurde ca. 1 ml einer Lösung von 4,4'-Dibrombiphenyl A (26,86 g, 0,0861 mol) gelöst in THF (25 ml) unter Stickstoff-Atmosphäre zugegeben. Die Mischung wurde erhitzt, um die Reaktion zu starten. Nach 20 Minuten wurde der Mischung ein weiterer Milliliter der Lösung zugegeben. Während der andauernden Wärmezufuhr wurde die restliche Lösung von 4,4'-Dibrombiphenyl A in THF tropfenweise über einen Zeitraum von 45 Minuten zugeführt. Nach der Zugabe der Lösung wurde die Mischung unter zeitgleichem Rückfluss von THF erhitzt.

Der Verbrauch des Anfangsmaterials wurde mittels Gaschromatographie (GC) geprüft. THF und gebildetes Salz wurde entfernt. Durch Destillation unter reduziertem Druck wurden 16,78 g (0,049 mol) - entsprechend einer Ausbeute von 57 % - 4,4'- Bis(ethoxydimethylsilyl)-biphenyl A als farblose transparente Flüssigkeit erhalten.

2. Schritt: Synthese von 4,4'- Bis(vinyldimethylsilyl)biphenyl

In einem Glaskolben wurden 17,54 g (51 mmol) 4,4'- Bis(ethoxydimethylsilyl)-biphenyl 10 ml THF zugesetzt. Anschließend wurden der Mischung im Glaskolben unter Stickstoff-Atmosphäre 108 ml (108 mmol) einer 1,0 M Lösung von $CH_2$= CHMgBr in THF tropfenweise bei Zimmertemperatur über eine Zeit von 30 Minuten zugegeben. Nach Zugabe der Grignard Verbindung in THF wurde über den Zeitraum von 5 Stunden am Rückfluss erhitzt. Nachdem die Mischung über Nacht bei Zimmertemperatur inkubiert wurde, wurde der Verbrauch des Anfangsmaterials und die Bildung des gewünschten Produktes mittels Gaschromatographie (GC) geprüft.

Zu der gewonnenen Mischung wurden 10 ml Methanol zugegeben, um überschüssiges $CH_2$= CHMgBr zu zersetzen, sowie THF und gebildetes Salz entfemt. Durch Destillation unter reduziertem Druck wurde die Verbindung als farblose transparente Flüssigkeit isoliert. Die Ausbeute betrug ca. 30 -50 %.

**Beispiel 1 (erfindungsgemäß)**

In einem geschlossenen Kneter wurden 36 Teile der Verbindung aus Synthesebeispiel 1 mit 61 Teilen Quarzmehl mit einer mittleren Korngröße von 10 µm, zwei Teilen einer pyrogen hergestellten, hochdispersen, hydrophobierten Kieselsäure mit einer BET-Oberfläche von 170 $m^2/g$ und einem Teil eines Platin-Katalysators mit einem Gehalt an reinem Platin von 1 % für 1,5 Stunden homogenisiert und danach 15 Minuten im Vakuum entgast.

Es wurde eine mittelfließende Paste (ISO 4823) erhalten. Die Paste stellt die Komponente A des erfindungsgemäßen Zweikomponenten-Siliconmaterials gemäß eines ersten Ausführungsbeispiels dar. Nach einer Lagerung bei 23 °C über einen Monat waren die Viskosität und die Reaktivität im Sollbereich.

**Beispiel 2 (erfindungsgemäß)**

In einem geschlossenen Kneter wurden 6 Teile der Verbindung aus Synthesebeispiel 1 mit 30 Teilen eines Polymethylhydrogensiloxans mit einer Viskosität von 30 mPa·s (gemessen bei 20°C) sowie einem SiH-Gehalt von 9,3 mmol/g (Silopren® U930), 63 Teilen Quarzmehl mit einer mittleren Korngröße von 10 µm und einem Teil einer pyrogen hergestellten, hochdispersen, hydrophobierten Kieselsäure mit einer BET-Oberfläche von 170 $m^2/g$ für 1,5 Stunden homogenisiert und danach 15 Minuten im Vakuum entgast.

Es wurde eine mittelfließende Paste (ISO 4823) erhalten. Die Paste stellt die Komponente B des erfindungsgemäßen Zweikomponenten-Siliconmaterials dar. Nach einer Lagerung bei 23 °C über einen Monat waren die Viskosität und die Reaktivität im Sollbereich.

**Beispiel 3 (erfindungsgemäß)**

50 Teile der in Beispiel 1 beschriebenen Komponente A und 50 Teile der in Beispiel 2 beschriebenen Komponente B wurden aus einer Kartusche (Fa. Mixpac) ausgedrückt und über einen statischen Mischer (Fa. Mixpac) homogen gemischt.

Das Produkt blieb ca. 60 Sekunden bei Raumtemperatur verarbeitbar und härtete bei einer Temperatur von 35 °C innerhalb von etwa drei Minuten nach Mischbeginn vollständig aus. Als Vulkanisat wurden harte, schwer komprimierbare Formkörper erhalten, welche fräsbar waren.

Nach 24 Stunden Lagerung der Probekörper in Wasser bei 37°C wurden folgende mechanischen Eigenschaften ermittelt:

| | |
|---|---|
| Shore D-Härte (DIN 53505): | 70 |
| Elastizitätsmodul im 3-Punkt-Biegeversuch (ISO 10477): | 902 MPa |
| Biegefestigkeit (ISO 10477): | 20,7 MPa |

Die zuvor genannten Messwerte sind jeweils Mittelwerte aus der Prüfung von fünf Prüfkörpern.

Das Elastizitätsmodul im 3-Punkt-Biegeverversuch wurde als Sekantenmodul zwischen 0,05% und 0,25% Biegung nach folgender Formel berechnet:

$$\text{E-Modul} = [L_V{}^3 * (X_H - X_L)] / (4 * \text{delta-L} * b_0 * a_0{}^3)$$

Die Biegefestigkeit wurde nach folgender Formel berechnet:

$$\text{Biegefestigkeit} = [(1{,}5 * L_V) / (b_0 * a_0{}^2)] * F$$

wobei:

| | |
|---|---|
| $a_0$: | Probendicke |
| $b_0$ : | Probenbreite |
| $L_V$: | Stützweite |
| $X_H$: | Kraft bei Ende der E-Modul-Ermittlung |
| $X_L$: | Kraft zu Beginn der E-Modul-Ermittlung |
| delta-L: | Durchbiegung zwischen $X_H$ und $X_L$ und |
| F: | Kraft bei Bruch der Probe |

Als Probekörper wurde eine Flach-/Streifenprobe mit folgenden Abmessungen verwendet:

Probendicke $a_0$ = 2 mm, Probenbreite $b_0$ = 2 mm, Probenlänge = 25 mm.
Die Stützweite betrug Lv = 20 mm.

**Beispiel 4 (erfindungsgemäß)**

In einem geschlossenen Kneter wurden 36 Teile der Verbindung aus Synthesebeispiel 2 mit 61 Teilen Quarzmehl mit einer mittleren Korngröße von 0,5 μm, zwei Teilen einer pyrogen hergestellten, hochdispersen, hydrophobierten Kieselsäure mit einer BET-Oberfläche von 170 $m^2/g$ und einem Teil eines Platin-Katalysators mit einem Gehalt an reinem Platin von 1 %, für 1,5 Stunden homogenisiert und danach 15 Minuten im Vakuum entgast.

Es wurde eine mittelfließende Paste (ISO 4823) erhalten. Die Paste stellt die Komponente A des erfindungsgemäßen Zweikomponenten-Siliconmaterials gemäß eines zweiten Ausführungsbeispiels dar. Nach einer Lagerung bei 23 °C über einen Monat waren die Viskosität und die Reaktivität im Sollbereich.

**Beispiel 5 (erfindungsgemäß)**

In einem geschlossenen Kneter wurden 33 Teile eines Polymethylhydrogensiloxans mit einer Viskosität von 30 mPa·s (gemessen bei 20°C) sowie einem SiH-Gehalt von 9,3 mmol/g mit 65 Teilen Quarzmehl mit einer mittleren Korngröße von 0,5 μm und zwei Teilen einer pyrogen hergestellten, hochdispersen, hydrophobier ten Kieselsäure mit einer BET-Oberfläche von 170 $m^2/g$ für 1,5 Stunden homogenisiert und danach 15 Minuten im Vakuum entgast.

Es wurde eine mittelfließende Paste (ISO 4823) erhalten. Die Paste stellt die Komponente B des erfindungsgemäßen Zweikomponenten-Siliconmaterials dar. Nach einer Lagerung bei 23 °C über einen Monat waren die Viskosität und die Reaktivität im Sollbereich.

**Beispiel 6 (erfindungsgemäß)**

50 Teile der in Beispiel 4 beschriebenen Komponente A und 50 Teile der in Beispiel 5 beschriebenen Komponente B wurden aus einer Kartusche (Fa. Mixpac) ausgedrückt und über einen statischen Mischer (Fa. Mixpac) homogen gemischt.

Das Produkt blieb ca. 60 Sekunden bei Raumtemperatur verarbeitbar und härtete bei einer Temperatur von 35 °C innerhalb von etwa drei Minuten nach Mischbeginn vollständig aus. Als Vulkanisat wurden harte, schwer komprimierbare Formkörper erhalten, welche fräsbar waren.

Nach 24 Stunden Lagerung der Probekörper in Wasser bei 37°C wurden folgende mechanischen Eigenschaften ermittelt:

| | |
|---|---|
| Shore D-Härte (DIN 53505): | 75 |
| Elastizitätsmodul im 3-Punkt-Biegeversuch (ISO 10477): | 1.074 MPa |
| Biegefestigkeit (ISO 10477): | 19,4 MPa |

**Beispiel 7 (erfindungsgemäß)**

In einem geschlossenen Kneter wurden 35 Teile der Verbindung aus Synthesebeispiel 3 mit 64 Teilen Quarzmehl mit einer mittleren Korngröße von 3 µm, zwei Teilen einer pyrogen hergestellten, hochdispersen, hydrophobierten Kieselsäure mit einer BET-Oberfläche von 170 $m^2/g$ und einem Teil eines Platin-Katalysators mit einem Gehalt an reinem Platin von 1 %, für 1,5 Stunden homogenisiert und danach 15 Minuten im Vakuum entgast.

Es wurde eine mittelfließende Paste (ISO 4823) erhalten. Die Paste stellt die Komponente A des erfindungsgemäßen Zweikomponenten-Siliconmaterials dar. Nach einer Lagerung bei 23 °C über einen Monat waren die Viskosität und die Reaktivität im Sollbereich.

**Beispiel 8 (erfindungsgemäß)**

In einem geschlossenen Kneter wurden 10 Teile der Verbindung aus Synthesebeispiel 3, 67 Teile Quarzmehl mit einer mittleren Korngröße von 3 µm und zwei Teilen einer pyrogen hergestellten, hochdispersen, hydrophobierten Kieselsäure mit einer BET-Oberfläche von 170 $m^2/g$ mit 21 Teilen eines Polymethylhydrogensiloxans mit einer Viskosität von 30 mPa·s (gemessen bei 20°C) und einem SiH-Gehalt von 9,3 mmol/g für 1,5 Stunden homogenisiert und danach 15 Minuten im Vakuum entgast.

Es wurde eine mittelfließende Paste (ISO 4823) erhalten. Die Paste stellt die Komponente B des erfindungsgemäßen Zweikomponenten-Siliconmaterials dar. Nach einer Lagerung bei 23 °C über einen Monat waren die Viskosität und die Reaktivität im Sollbereich.

**Beispiel 9 (erfindungsgemäß)**

50 Teile der in Beispiel 7 beschriebenen Komponente A und 50 Teile der in Beispiel 8 beschriebenen Komponente B wurden aus einer Kartusche (Fa. Mixpac) ausgedrückt und über einen statischen Mischer (Fa. Mixpac) homogen gemischt.

Das Produkt blieb ca. 60 Sekunden bei Raumtemperatur verarbeitbar und härtete bei einer Temperatur von 35 °C innerhalb von etwa zwei Minuten nach Mischbeginn vollständig aus. Als Vulkanisat wurden harte, schwer komprimierbare Formkörper erhalten, welche fräsbar waren.

Nach 24 Stunden Lagerung der Probekörper in Wasser bei 37°C wurden folgende mechanischen Eigenschaften ermittelt:

| | |
|---|---|
| Shore D-Härte (DIN 53505): | 83 |
| Elastizitätsmodul im 3-Punkt-Biegeversuch (ISO 10477): | 2.226 MPa |
| Biegefestigkeit (ISO 10477): | 31,7 MPa |

**Vergleichsbeispiel 1 (nicht erfindungsgemäß)**

Ein handelsübliches Bissregistrierungsmaterial auf Basis additionsvernetzender Vinylpolysiloxane wurde entsprechend der Herstelleranweisung gemischt und zur Abbindung gebracht.

Das Produkt blieb ca. 30 Sekunden bei Raumtemperatur verarbeitbar und härtete bei einer Temperatur von

35°C innerhalb von etwa zwei Minuten nach Mischbeginn vollständig aus.

Als Vulkanisat wurden Formkörper erhalten, welche gut fräsbar waren.

Nach 24 Stunden Lagerung der Probekörper in Wasser bei 37°C wurden folgende mechanischen Eigenschaften ermittelt:

| | |
|---|---|
| Shore D-Härte (DIN 53505): | 43 |
| Elastizitätsmodul im 3-Punkt-Biegeversuch (ISO 10477): | 38 MPa |
| Biegefestigkeit (ISO 10477): | 8,7 MPa |

Die Bestimmung und Berechnung des Elastizitätsmoduls im 3-Punkt-Biegeversuch und der Biegefestigkeit erfolgte wie in Beispiel 3 beschrieben.

Dieses Beispiel verdeutlicht, dass Bissregistriermaterialien auf Basis additionsvernetzender Vinylpolysiloxane nach dem Stand der Technik über eine merklich geringere Härte bzw. ein signifikant geringeres Elastizitätsmodul verfügen.

Die Ergebnisse der einzelnen Beispiele und Vergleichsbeispiele sind in Tabelle 1 zusammengefasst.

**Tabelle 1**

| Beispiel | E-Modul (1) im 3-Punkt-Biegeversuch | Biegefestigkeit(1) | Shore D Härte (1) | Shore A Härte (1) | Abbindezeit |
|---|---|---|---|---|---|
| Bsp. 3 | 902 MPa | 20,7 MPa | 70 | > 95 | 3,0 min |
| Bsp. 6 | 1.074 MPa | 19,4 MPa | 75 | > 95 | 3,0 min |
| Bsp. 9 | 2.226 MPa | 31,7 MPa | 83 | > 95 | 2,0 min |
| V.bsp. 1 | 38 MPa | 8,7 MPa | 43 | > 95 | 2,0 min |
| Bsp.: Beispiel<br>V.bsp.: Vergleichsbeispiel<br>(1) nach 24 h bei 37°C | | | | | |

**Patentansprüche**

**1.** Über Hydrosilylierung additionsvemetzendes Zweikomponenten-Dentalmaterial enthaltend

a) wenigstens eine Verbindung mit Vinylgruppen im Molekül,
b) wenigstens eine Organohydrogensiliziumverbindung und
c) wenigstens einen Katalysator,

**dadurch gekennzeichnet, dass** das Dentalmaterial im ausvulkanisierten Zustand eine Shore D-Härte (nach DIN 53505) von mehr als 69 und/oder eine Biegefestigkeit von mindestens 8 MPa und/oder einen Elastizitätsmodul im Biegeversuch, gemessen nach ISO 10477 von mindestens 300 MPa, aufweist, und dass die wenigstens eine Verbindung a) und/oder die wenigstens eine Verbindung b)
als erste Struktureinheit wenigstens zumindest eine voluminöse und/oder starre Gruppe ausgewählt aus der Gruppe der tertiären Alkyl-, quartären Alkyl-, Cycloalkyl-, Cycloalkenyl-, Aryl-, Aralkyl-, Alkylarylgruppen, halogensubstituierten tertiären Alkylgruppen, halogensubstituierten quartären Alkylgruppen, halogenierten Arylgruppen, halogenierten Aralkylgruppen, halogenierten Alkylarylgruppen, besonders bevorzugt aromatischen und nichtaromatischen Mono-, Bis-, Oligo-, Polycyclusgruppen, Bisphenol A-, Bisphenol B-, Bisphenol F-Gruppen, 1,1,1-Tris(4-hydroxyphenyl)alkan-Gruppen, Norbornan-Gruppen, Adamantan-Gruppen und Pentaerythrit-Gruppen, umfasst, sowie als zweite Struktureinheit wenigstens zwei alkenylfunktionelle und/oder wenigstens zwei, bevorzugt wenigstens drei wasserstofffunktionelle Silyleinheiten der allgemeinen Formeln I umfasst

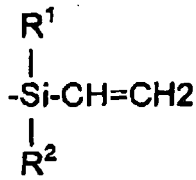 (Ia) und/oder 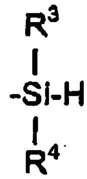 (Ib)

mit $R^1$, $R^2$ unabhängig voneinander ausgewählt aus der Gruppe, welche aus Alkylgruppen, Alkenylgruppen, Arylgruppen, Aralkylgruppen, Alkylarylgruppen, halogenierten Alkylgruppen, halogenierten Arylgruppen, halogenierten Aralkylgruppen, halogenierten Alkylarylgruppen, Cyanoalkylgruppen, Siloxygruppen, Cyloalkylgruppen und Cycloalkenylgruppen besteht, wobei Alkylgruppen besonders und Methylgruppen ganz besonders bevorzugt sind, und $R^3$, $R^4$ unabhängig voneinander H oder $R^1$, wobei Alkylgruppen besonders und Methylgruppen ganz besonders bevorzugt sind,
wobei die zweite Struktureinheit

i) direkt,
ii) über ein Sauerstoffatom,
iii) über eine Spacergruppe ausgewählt aus der Gruppe $-O-CH_2CH_2-$, $-O-(CH_2)_4-$, $-O-CR^1R^2-CR^1R^2-$, $-CH_2CH_2CH_2(OCH_2CH_2)_4-$, $-CH_2-CH_2-CH_2-$, $-O-C(CH_3)_2-CH_2-$, $-O-CH(CH_3)-CH_2-$, $-O-CH(C_2H_5)-CH_2-$, $-O-CH(C_4H_9)-CH_2-$, und $-O-CH-(C_{10}H_{21})-CH_2-$, worin $R^1$ und $R^2$ unabhängig voneinander die oben definierte Bedeutung besitzen, oder
iv) über ein Sauerstoffatom kombiniert mit einer Spacergruppe gemäß iii),

an die erste Struktureinheit gebunden ist und wobei die wenigstens eine Verbindung mit Vinylgruppen im Molekül a) einen Si-Vinylgehalt von 2 bis 10 mmol/g aufweist und die wenigstens eine Organohydrogensiliziumverbindung b) einen Si-H Gehalt von 4-15 mmol / g aufweist.

2. Dentalmaterial nach Anspruch 1, **dadurch gekennzeichnet, dass** der Abstand der wenigstens zwei Vinylgruppen der wenigstens einen Verbindung a) voneinander und/oder der Abstand der wenigstens zwei SiH-Gruppen der wenigstens einen Verbindung b) voneinander wenigstens 0,3 nm, besonders bevorzugt wenigstens 0,45 nm, ganz besonders bevorzugt wenigstens 0,5 nm und höchst besonders bevorzugt wenigstens 0,7 nm beträgt.

3. Dentalmaterial nach Anspruch 1 oder 2, **dadurch gekennzeichnet, dass** die erste Struktureinheit der wenigstens einen Verbindung a) und/oder der wenigstens einen Verbindung b) ein aromatischer Mono-, Bis-, Oligo- oder Polycyclus und/oder ein nichtaromatischer Mono-, Bis-, Oligo- oder Polycyclus, besonders bevorzugt eine Bisphenol A-, Bisphenol B-, Bisphenol F-Gruppe, 1,1,1-Tris(4-hydroxyphenyl)alkan-Gruppe, Norbornan-Gruppe, Adamantan-Gruppe oder Pentaerythrit-Gruppe, ist.

4. Dentalmaterial nach einem der vorhergehenden Ansprüche, **dadurch gekennzeichnet, dass** die erste Strukturеinheit der wenigstens einen Verbindung a) und/oder der wenigstens einen Verbindung b) ausgewählt aus der Gruppe, welche aus

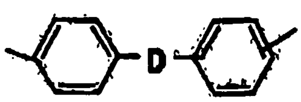 , 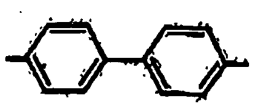 , 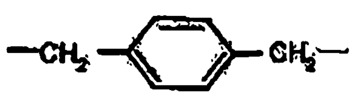

o, m, p 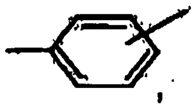 , 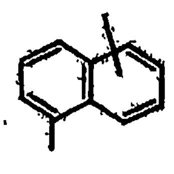 , 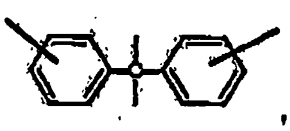 , 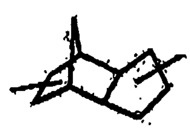

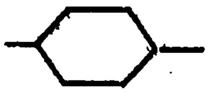 , 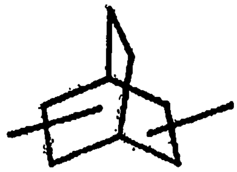 , 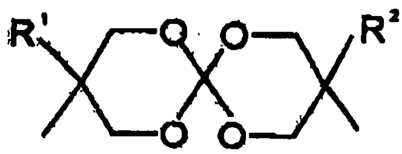

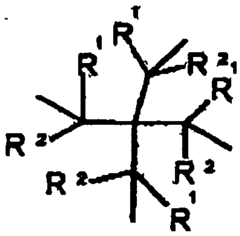 , 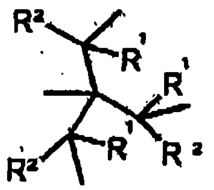 , 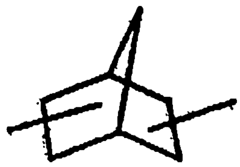

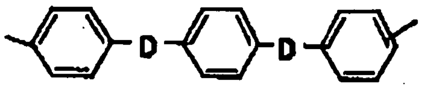 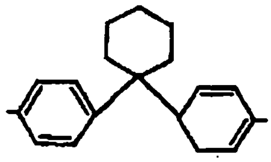

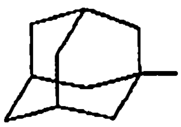 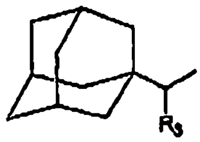 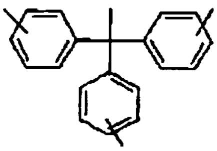

worin $R^1$, $R^2$, $R^3$ wie in den Formeln I definiert sind,
D= $CR^3R^4$ mit $R^3$ und $R^4$ in Formel Ib definiert oder Wasserstoff, insbesondere D gleich $CH_2$, $C(CH_3)_2$, CMePh mit Me=$CH_3$ und Ph=$C_6H_5$ oder $CPh_2$
besteht, ist.

5. Dentalmaterial nach einem der vorhergehenden Ansprüche, **dadurch gekennzeichnet, dass** die erste Struktureinheit der wenigstens einen Verbindung a) und/oder der wenigstens einen Verbindung b) Bisphenol A, ein Bisphenol A-Derivat oder eine Trisphenolverbindung gemäß einer der beiden allgemeinen Formeln

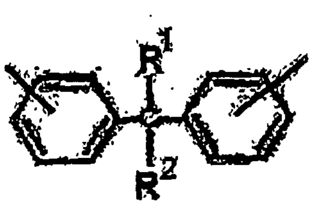 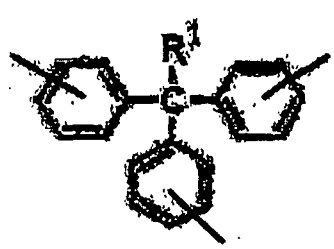

mit $R^1$, $R^2$ wie oben definiert
ist.

6. Dentalmaterial nach einem der vorhergehenden Ansprüche, **dadurch gekennzeichnet, dass** die wenigstens eine Verbindung a) und/oder die wenigstens eine Verbindung b) eine Substanz der allgemeinen Formeln II

$$H^2C{=}CH\text{-}SiR^1R^2\text{-}E_{n1}\text{-}SiR^1R^2\text{-}CH{=}CH_2 \qquad \text{(IIa)}$$

und/oder

$$H\text{-}SiR^3R^4\text{-}E_{n1}\text{-}SiR^3R^4\text{-}H \qquad \text{(IIb)}$$

wobei die Reste $R^1$, $R^2$, $R^3$, $R^4$ wie in Formel I definiert sind, E die erste Struktureinheit bezeichnet und $n_1$ eine ganze Zahl ≥ 1, vorzugsweise 1, ist,
ist/enthält.

7. Dentalmaterial nach einem der vorhergehenden Ansprüche, **dadurch gekennzeichnet, dass** die wenigstens eine Verbindung a) und/oder die wenigstens eine Verbindung b) eine Substanz der allgemeinen Formeln III

$$H_2C{=}CH\text{-}SiR^1R^2\text{-}O\text{-}E_{n1}\text{-}O\text{-}SiR^1R^2\text{-}CH{=}CH_2 \qquad \text{(IIIa).}$$

und/oder

$$H\text{-}SiR^3R^4\text{-}O\text{-}E_{n1}\text{-}O\text{-}SiR^3R^4\text{-}H \qquad \text{(IIIb)}$$

wobei die Reste $R^1$, $R^2$, $R^3$, $R^4$ und E sowie $n_1$ wie oben definiert sind,
ist/enthält.

8. Dentalmaterial nach einem der vorhergehenden Ansprüche, **dadurch gekennzeichnet, dass** die wenigstens eine Verbindung a) und/oder die wenigstens eine Verbindung b) eine Substanz der allgemeinen Formeln IV

$$H_2C{=}CH\text{-}SiR^1R^2\text{-}A^1{}_{n2}\text{-}E_{n1}\text{-}A^2{}_{n3}\text{-}SiR^1R^2\text{-}CH{=}CH_2 \qquad \text{(IVa)}$$

und/oder

$$H\text{-}SiR^3R^4\text{-}A^1{}_{n2}\text{-}E_{n1}\text{-}A^2{}_{n3}\text{-}SiR^3R^4\text{-}H\ , \qquad \text{(IVb)}$$

wobei die Reste $R^1$, $R^2$, $R^3$, $R^4$ und E sowie $n_1$ wie oben definiert sind, $n_2$, $n_3$ gleich oder verschieden und jeweils ganze Zahlen ≥ 1, vorzugsweise 1, sind sowie $A^1$, $A^2$ unabhängig voneinander ein Spacer gemäß Anspruch 1 ist,
ist/enthält,

9. Dentalmaterial nach einem der vorhergehenden Ansprüche, **dadurch gekennzeichnet, dass** die wenigstens eine Verbindung a) und/oder die, wenigstens eine Verbindung b) aus wenigstens einer der genannten ersten sowie wenigstens einer der genannten zweiten Struktureinheit besteht, wobei die wenigstens eine zweite Struktureinheit über einen Spacer gemäß Anspruch 1, welcher über ein Sauerstoffatom an die wenigstens eine erste Struktureinheit gebunden ist, mit der ersten Struktureinheit verbunden ist.

10. Dentalmaterial nach einem der vorhergehenden Ansprüche, **dadurch gekennzeichnet, dass** die wenigstens eine Verbindung a) und/oder die wenigstens eine Verbindung b) genau eine starre und/oder volumöse Gruppe aufweist.

11. Dentalmaterial nach einem der vorhergehenden Ansprüche, **dadurch gekennzeichnet, dass** dieses im ausvulkanisierten Zustand eine Biegefestigkeit von mindestens 15 MPa und ganz besonders bevorzugt mindestens 19 MPa und/oder ein Elastizitätsmodul im Biegeversuch (gemessen nach ISO 10477) von mindestens 600 MPa und ganz besonders bevorzugt mindestens 900 MPa, aufweist.

12. Dentalmaterial nach einem der vorhergehenden Ansprüche, **dadurch gekennzeichnet, dass** die mindestens eine Verbindung a) und die mindestens eine Verbindung b) jeweils eine erste und zweite Struktureinheit gemäß Anspruch 1 enthalten.

13. Dentalmaterial nach einem der vorhergehenden Ansprüche, **dadurch gekennzeichnet, dass** dieses neben mindestens einer Verbindung a) und/oder mindestens einer Verbindung b) mit jeweils einer ersten und zweiten Struktureinheit gemäß Anspruch 1 ein oder mehrere Organohydrogenpolysiloxane, besonders bevorzugt solche mit wenigstens zwei Si-H-Gruppen pro Molekül und einem Si-H-Gehalt zwischen 4 und 14 und ganz besonders bevorzugt zwischen 5 und 13 mmol/g, sowie einer Viskosität (bei 20°C) von 5 bis 2.000 mPa·s, enthalten.

**14.** Dentalmaterial nach einem der vorhergehenden Ansprüche, **dadurch gekennzeichnet, dass** die wenigstens eine Organohydrogensiliziumverbindung b) eine Verbindung der allgemeinen Formel VI

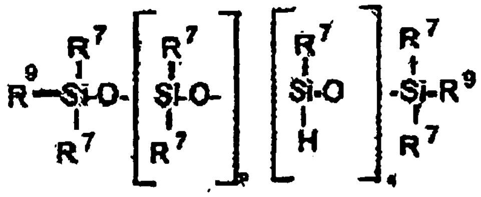

mit p = 0 bis 1500
mit q = 0 bis 1500, bevorzugt 2 bis 1500

mit $R^7$ = Alkyl- (z.B. Methyl-, Ethyl-, Isopropyl-), Aryl- (z.b. Phenyl-, Naphtyl-, Tolyl-, Xylyl-), Aralkyl- (Benzyl-, Phenylethyl-) und halogensubstitulerte Alkyl-und Aryl-Gruppen (z.B.3.3.3-Trifluorpropyl-, Chlorphenyl-, Difluorphanyl-), Cyanalkyl-, Cycloalkyl- und Cycloalkenyl-. Vorzugweise ist R = Methyl-.
mit $R^9$ = $R^7$ und/oder H

mit der Maßgabe, dass wenigstens 2 Si-Atome der Formel VI ein H-Atom tragen,
ist.

**15.** Dentalmaterial nach einem der vorhergehenden Ansprüche, **dadurch gekennzeichnet, dass** die wenigstens eine Organohydrogensiliziumverbindung b) eine Verbindung der allgemeinen Formel VII

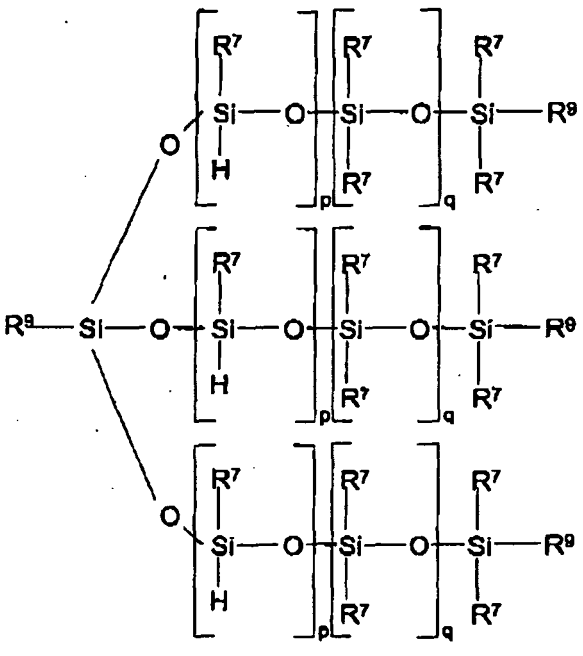

worin die Reste die in der Formel VI angegebene Bedeutung haben
ist.

**16.** Dentalmaterial nach einem der vorhergehenden Ansprüche, **dadurch gekennzeichnet, dass** die wenigstens eine Organohydrogensiliziumverbindung b) eine Verbindung der allgemeinen Formel VIII

$$[SiO_{4/2}]\ [SiO_{1/2}R^1R^2H]_m$$

mit m=0,9 bis 4, bevorzugt m=1 bis 4 ist.

**17.** Dentalmaterial nach einem der vorhergehenden Ansprüche, **dadurch gekennzeichnet, dass** die wenigstens eine Organohydrogensiliziumverbindung b) ein Si-H-haltiges MQ-Harz bestehend aus $(R^9)_3SiO_{1/2}$, $(R^7)_2(H)SiO_{1/2}$ und $SiO_{4/2}$-Einheiten, wobei auch noch als T-Einheiten das trifunktionelle $(R^9)_1SiO_{3/2}$ und als D-Einheiten das bifunktionelle $R^7R^9SiO_{2/2}$, wobei die Reste $R^7$ und $R^9$ die zuvor bezeichneten Bedeutungen haben, vorhanden sein können, ist.

**18.** Dentalmaterial nach einem der vorhergehenden Ansprüche, **dadurch gekennzeichnet, dass** die wenigstens eine Organohydrogensiliziumverbindung b) umfassend eine erste und zweite Struktureinheit nach Anspruch 1 einen SiH-Gehalt von 4 bis 15 mmol/g und ganz besonders bevorzugt von 7 bis 15 mmol/g sowie eine Viskosität (bei 20°C) von 1 bis 10.000 mPa·s, bevorzugt von 5 bis 2.000 mPa·s, aufweist.

**19.** Dentalmaterial nach einem der vorhergehenden Ansprüche, **dadurch gekennzeichnet, dass** es

$a_1$) 1 bis 90 Gew.-%, besonders bevorzugt 5 bis 75 Gew.-% und ganz beisonders bevorzugt 10 bis 60 Gew.-% wenigstens einer Verbindung a) mit mindestens einer starren und/oder voluminösen Gruppe sowie wenigstens zwei vinylfunktionellen Silylgruppen im Molekül und einem Si-Vinylgehalt von 2 bis 10 mmol/g,
$a_2$) 0 bis 40 Gew.-%, besonders bevorzugt 0 bis 30 Gew.-% und ganz besonders bevorzugt 0 bis 20 Gew.-% wenigstens eines Organopolysiloxans mit mindestens zwei Vinylgruppen im Molekül und einem Si-Vinylgehalt von 0,5 bis 10 mmol/g, bevorzugt einem Si-Vinylgehalt von 1 bis 10 mmol/g und besonders bevorzugt einem Si-Vinylgehalt von 2 bis 10 mmol/g, sowie einer Viskosität von 21 bis 350.000 mPa·s,
$b_1$) 0 bis 90 Gew.-%, besonders bevorzugt 5 bis 75 Gew.-% und ganz besonders bevorzugt 10 bis 60 Gew.-% einer Organohydrogenslliziumverbindung mit mindestens einer starren und/oder voluminösen Gruppe sowie wenigstens zwei Silylgruppen mit mindestens zwei, bevorzugt mindestens 3 SiH-Gruppen und einem SiH-Gehalt von 4 bis 15 mmol/g und besonders bevorzugt von 7 bis 15 mmol/g.
$b_2$) 0 bis 50 Gew.-%, besonders bevorzugt 0 bis 40 Gew.-% und ganz besonders bevorzugt 0 bis 30 Gew.-% eines Organohydrogenpolysiloxans mit mindestens zwei, bevorzugt mindestens 3 SiH-Gruppen, und einem SiH-Gehalt von 0,1 bis 15 mmol/g, bevorzugt von 4 bis 15 mmol/g, besonders bevorzugt von 7 bis 15 mmol/g und ganz besonders bevorzugt von 7 bis 13 mmol/g, sowie einer Viskosität zwischen 5 und 2.000 mPa·s, wobei die Summe der Gewichtsprozente der Verbindungen $b_1$) und $b_2$) mindestens 1 Gew.-% beträgt,
c) 0,00001 bis 0,2 Gew.-%, vorzugsweise 0,0005 bis 0,1 Gew.-% wenigstens eines Katalysators zur Beschleunigung der Hydrosilylierungsreaktion, bezogen auf reines Metall,
d) 0 bis 50 Gew.-%, besonders bevorzugt 0,1 bis 40 Gew.-% und ganz besonders bevorzugt 0,5 bis 35 Gew.-% an verstärkenden Füllstoffen mit einer BET-Oberfläche von mindestens 50 $m^2$/g,
e) 0 bis 90 Gew.-%, besonders bevorzugt 0 bis 80 Gew.-% und ganz besonders bevorzugt 0 bis 75 Gew.-% an nicht verstärkenden Füllstoffen mit einer BET-Oberfläche von weniger als 50 $m^2$/g und einer mittleren Korngröße von wenigstens 0,1 $\mu$m,
f) 0 bis 5 Gew.-%, vorzugsweise 0 bis 2 Gew.-% wenigstens eines Farbstoffes,
g) 0 bis 30 Gew.-%, vorzugsweise 0 bis 5 Gew.-% an Feuchtigkeitsbinder,
h) 0 bis 1 Gew.-%, vorzugsweise 0 bis 0,6 und besonders bevorzugt 0 bis 0,1 Gew.-% an Inhibitoren,
i) 0 bis 40 Gew.-%, besonders bevorzugt 0 bis 30 Gew.-% und ganz besonders bevorzugt 0 bis 20 Gew.-% wenigstens eines vinylgruppenhaltigen und/oder SiH-haltigen und/oder SiOR-haltigen (mit R=H oder Alkyl) QM-Harzes mit einem SiH-Gehalt von 0 bis 15 mmol/g, besonders bevorzugt von 0 bis 1 mmol/g und/oder einem SiOR-Gehalt von 0 bis 0,5 mmol/g,
j) 0 bis 80 Gew.-%, vorzugsweise 0 bis 50 Gew.-% an Compounds aus vinylgruppenhaltigen Organopolysiloxanen und verstärkenden Füllstoffen,
k) 0 bis 10 Gew.-%, vorzugsweise 0 bis 5 Gew.-% an Tensiden, Emulgatoren und/oder Stabilisatoren,
l) 0 bis 90 Gew.-%, vorzugsweise 0 bis 80 Gew.-% an radioopaken Substanzen,
m) 0 bis 20 Gew.-%, vorzugsweise 0 bis 10 Gew.-% an $H_2$-Absorbern/Adsorbern oder an die $H_2$-Entwicklung reduzierenden bzw. eliminierenden Substanzen, sowie
n) 0 bis 20 Gew.-%, vorzugsweise 0 bis 15 Gew.-% an weiteren Hilfs- und Zusatzstoffen
enthält.

**20.** Verwendung eines Dentalmaterials nach einem der Ansprüche 1 bis 19 zur Herstellung von Bissregistriermaterial, Zement, provisorischem Kron- und Brückenmaterial, provisorischem Füllmaterial und/oder dauerhaftem Füllmaterial.

## Claims

1. Via hydrosilylation addition-crosslinking two-component dental material comprising

a) at least one compound having vinyl groups in the molecule;
b) at least one organohydrogensilicon compound; and
c) at least one catalyst;
**characterized in that** said dental material has in the fully cured state a Shore D hardness (in accordance with DIN 53505) of greater than 69 and/or a flexural strength of at least 8 MPa and/or a modulus of elasticity in the flexural test measured in accordance with ISO 10477 of at least 300 MPa, and said at least one compound a) and/or said at least one compound b) comprises
as first repeating unit at least one bulky and/or rigid group selected from the group of tertiary alkyl, quaternary alkyl, cycloalkyl, cycloalkenyl, aryl, aralkyl, alkylaryl groups, halogen-substituted tertiary alkyl groups, halogen-substituted quarternary alkyl groups, halogenated aryl groups, halogenated aralkyl groups, halogenated alkylaryl groups, particularly preferably aromatic and non-aromatic mono-, bis-, oligo-, polycyclic groups, bisphenol A, bisphenol B, bisphenol F groups, 1,1,1-tris(4-hydroxyphenyl)alkane groups, norbornane groups, adamantane groups, and pentaerythritol groups, and
as second repeating unit at least two alkenyl-functional and/or at least two, preferably at least three hydrogen-functional silyl units of the general formula I

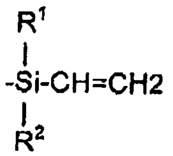 (Ia) and/or 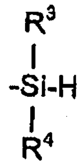 (Ib)

wherein $R^1$, $R^2$ are independently selected from the group consisting of alkyl groups, alkenyl groups, aryl groups, aralkyl groups, alkylaryl groups, halogenated alkyl groups, halogenated aryl groups, halogenated aralkyl groups, halogenated alkylaryl groups, cyanoalkyl groups, siloxy groups, cycloalkyl groups, and cycloalkenyl groups, alkyl groups being preferred and methyl groups being particularly preferred, and
$R^3$, $R^4$ are independently H or $R^1$, alkyl groups being preferred and methyl groups being particularly preferred, the second repeating unit being bonded

i) directly;
ii) via an oxygen atom;
iii) via a spacer group selected from the group of $-O-CH_2CH_2-$, $-O-(CH_2)_4-$, $-O-CR^1R^2-CR^1R^2-$, $-CH_2CH_2CH_2(OCH_2CH_2)_4-$, $-CH_2-CH_2-CH_2-$, $O-C(CH_3)_2-CH_2-$, $-O-CH(CH_3)-CH_2-$, $-O-CH(C_2H_5)-CH_2-$, $-O-CH(C_4H_9)-CH_2-$, and $-O-CH-(C_{10}H_{21})-CH_2-$, wherein $R^1$ and $R^2$ independently have the meaning defined above, or
iv) via an oxygen atom combined with a spacer group according to iii),
to the first repeating unit and said at least one compound having vinyl groups in the molecule a) having a Si-vinyl content of 2 to 10 mmol/g and said at least one organohydrogensilicon compound b) having a Si-H content of 4-15 mmol/g.

2. A dental material according to claim 1, **characterized in that** the distance from each other of the at least two vinyl groups of said at least one compound a) and/or the distance from each other of the at least two SiH groups of said at least one compound b) is at least 0.3 nm, preferably 0.45 nm, more preferably 0.5 nm, and most preferably 0.7 nm.

3. A dental material according to claim 1 or claim 2, **characterized in that** the first repeating unit of said at least one compound a) and/or of said at least one compound b) is an aromatic mono-, bis-, oligo-, or polycycle and/or a non-aromatic mono-, bis-, oligo-, or polycycle, particularly preferably a bisphenol A, bisphenol B, bisphenol F group, 1,1,1-tris(4-hydroxyphenyl)alkane group, norbornane group, adamantane group, or pentaerythritol group.

4. A dental material according to any one of the preceding claims, **characterized in that** the first repeating unit of said at least one compound a) and/or of said at least one compound b) is selected from the group consisting of

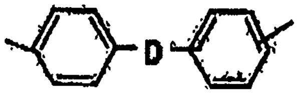 , 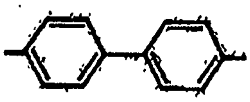 , 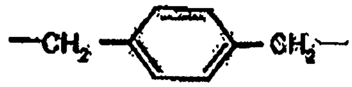

o, m, p 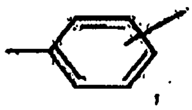 , 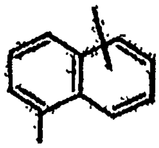 , 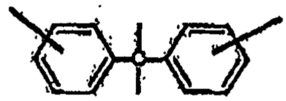 , 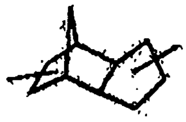

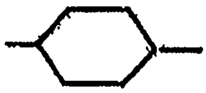 , 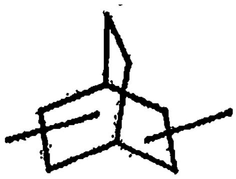 , 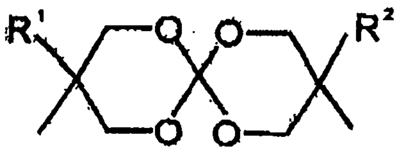

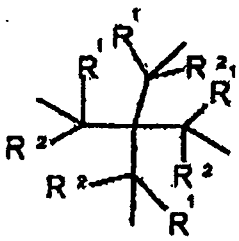 , 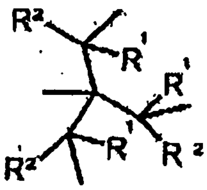 , 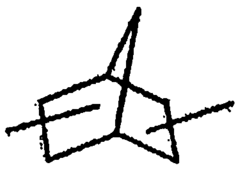

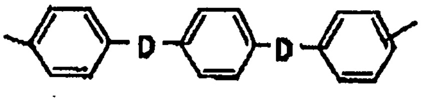 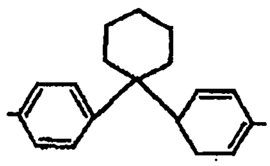

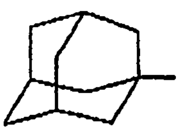 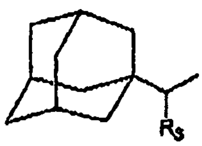 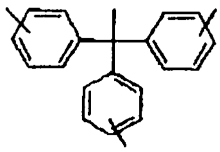

wherein $R^1$, $R^2$, $R^3$ are defined as in Formulas I, D = $CR^3R^4$ wherein $R^3$ and $R^4$ are as defined in Formula Ib or hydrogen, in particular D equals $CH_2$, $C(CH_3)_2$, CMePh with Me=$CH_3$ and Ph=$C_6H_5$ or $CPh_2$.

5. A dental material according to any one of the preceding claims, **characterized in that** the first repeating unit of said

at least one compound a) and/or of said at least one compound b) is bisphenol A, a bisphenol A derivative, or a trisphenol compound according to one of the two general formulas

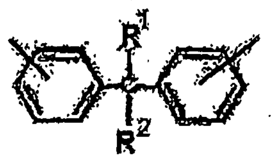

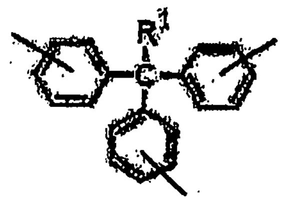

wherein $R^1$, $R^2$ are as defined above.

**6.** A dental material according to any one of the preceding claims, **characterized in that** said at least one compound a) and/or said at least one compound b) is/comprises a substance of the general formula II

$H_2C{=}CH{-}SiR^1R^2{-}E_{n1}{-}SiR^1R^2{-}CH{=}CH_2$ (IIa)

and/or

$H{-}SiR^3R^4{-}E_{n1}{-}SiR^3R^4{-}H$ (IIb),

wherein the radicals $R^1$, $R^2$, $R^3$, $R^4$ are defined as in Formula I, E refers to the first repeating unit, and n is an integer ≥ 1, preferably 1.

**7.** A dental material according to any one of the preceding claims, **characterized in that** said at least one compound a) and/or said at least one compound b) is/comprises a substance of the general formula III

$H_2C{=}CH{-}SiR^1R^2{-}O{-}E_{n1}{-}O{-}SiR^1R^2{-}CH{=}CH_2$ (IIIa)

and/or

$H{-}S1R^3R^4{-}O{-}E_{n1}{-}O{-}SiR^3R^4{-}H$ , (IIIb)

wherein the radicals $R^1$, $R^2$, $R^3$, $R^4$ and E and $n_1$ are defined as above.

**8.** A dental material according to any one of the preceding claims, **characterized in that** said at least one compound a) and/or said at least one compound b) is/comprises a substance of the general formula IV

$H_2C{=}CH{-}SiR^1R^2{-}A^1{}_{n2}{-}E_{n1}{-}A^2{}_{n3}{-}SiR^1R^2{-}CH{=}CH_2$ (IVa)

and/or

$H{-}SiR^3R^4{-}A^1{}_{n2}{-}E_{n1}{-}A^2{}_{n3}{-}SiR^3R^4{-}H$ (IVb) ,

wherein the radicals $R^1$, $R^2$, $R^3$, $R^4$ and E and $n_1$ are defined as above; $n_2$, $n_3$ are the same or different and each are integers ≥ 1, preferably 1; and $A^1$, $A^2$ are independently a spacer according to claim 1.

**9.** A dental material according to any one of the preceding claims, **characterized in that** said at least one compound a) and/or said at least one compound b) is composed of at least one of the mentioned first and at least one of the mentioned second repeating units, the at least one second repeating unit being linked to the first repeating unit via a spacer according to claim 1, which is bonded to the at least one first repeating unit via an oxygen atom.

**10.** A dental material according to any one of the preceding claims, **characterized in that** said at least one compound a) and/or said at least one compound b) has exactly one rigid and/or bulky group.

**11.** A dental material according to any one of the preceding claims, **characterized in that** said dental material has in the fully cured state a flexural strength of at least 15 MPa and particularly preferably at least 19 MPa and/or a modulus of elasticity in the flexural test (measured in accordance with ISO 10477) of at least 600 MPa and particularly preferably at least 900 MPa.

**12.** A dental material according to any one of the preceding claims, **characterized in that** said at least one compound a) and said at least one compound b) each comprise a first and second repeating unit according to claim 1.

**13.** A dental material according to any one of the preceding claims, **characterized in that** said dental material comprises, besides at least one compound a) and/or at least one compound b) each having a first and second repeating unit according to claim 1, one or more organohydrogenpolysiloxanes, particularly preferably those having at least two Si-H groups per molecule and a Si-H content between 4 and 14 and most preferably between 5 and 13 mmol/g, and a viscosity (at 20°C) of 5 to 2,000 mPa·s.

**14.** A dental material according to any one of the preceding claims, **characterized in that** said at least one organohydrogensilicon compound b) is a compound of the general formula VI

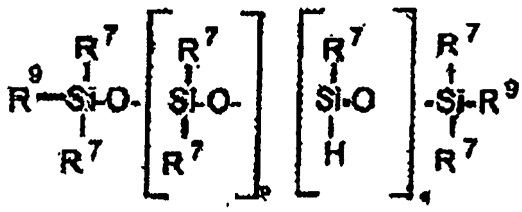

wherein p = 0 to 1500
wherein q = o to 1500, preferably 2 to 1500
wherein $R^7$ = alkyl (for example methyl, ethyl, isopropyl) , aryl (for example phenyl, naphtyl, tolyl, xylyl) , aralkyl (benzyl , phenylethyl) , and halogen-substituted alkyl and aryl groups (for example 3,3,3- trifluorpropyl, chlorophenyl, difluorphanyl), cyanalkyl, cycloalkyl, and cycloalkenyl. Preferably, R is methyl.
wherein $R^9$ = $R^7$ and/or H
with the proviso that at least two Si atoms of Formula VI carry an H atom.

**15.** A dental material according to any one of the preceding claims, **characterized in that** said at least one organohydrogensilicon compound b) is a compound of the general formula VII

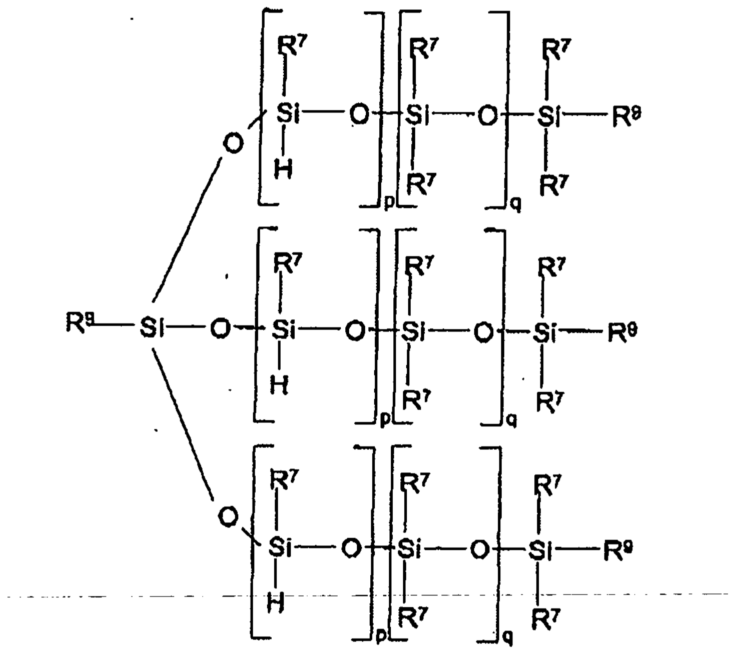

wherein the radicals have the meaning given in Formula VI.

**16.** A dental material according to any one of the preceding claims, **characterized in that** said at least one organohydrogensilicon compound b) is a compound of the general formula VIII

$$[SiO_{4/2}]\,[SiO_{1/2}R^1R^2H]_m$$

wherein m = 0.9 to 4, preferably m = 1 to 4.

**17.** A dental material according to any one of the preceding claims, **characterized in that** said at least one organohydrogensilicon compound b) is a Si-H-containing MQ resin consisting of $(R^9)_3SiO_{1/2}$, $(R^7)_2(H)SiO_{1/2}$, and $SiO_{4/2}$ units, wherein the trifunctional $(R^9)_1SiO_{3/2}$ can also be present as T units and the bifunctional $R^7R^9SiO_{2/2}$ can also be present as D units, the radicals $R^7$ und $R^9$ having the meaning previously described.

**18.** A dental material according to any one of the preceding claims, **characterized in that** said at least one organohydrogensilicon compound b) comprising a first and second repeating unit according to claim 1 has a SiH content of 4 to 15 mmol/g and most preferably from 7 to 15 mmol/g and a viscosity (at 20°C) of 1 to 10,000 mPa·s, preferably from 5 to 2,000 mPa·s.

**19.** A dental material according to any one of the preceding claims, **characterized in that** said dental material comprises

$a_1$) 1 to 90% by weight, more preferably 5 to 75% by weight, and most preferably 10 to 60% by weight of at least one compound a) having at least one rigid and/or bulky group and at least two vinyl- functional silyl groups in the molecule and a Si-vinyl content of 2 to 10 mmol/g;
$a_2$) 0 to 40% by weight, more preferably 0 to 30% by weight, and most preferably 0 to 20% by weight of at least one organopolysiloxane having at least two vinyl groups in the molecule and a Si-vinyl content of 0.5 to 10 mmol/g, preferably a Si-vinyl content of 1 to 10 mmol/g, and most preferably a Si-vinyl content of 2 to 10 mmol/g, and a viscosity of 21 to 350,000 mPa·s;
$b_1$) 0 to 90% by weight, more preferably 5 to 75% by weight, and most preferably 10 to 60% by weight of an organohydrogensilicon compound having at least one rigid and/or bulky group and at least two silyl groups having at least two, preferably at least three SiH groups and a SiH content of 4 to 15 mmol/g and most preferably 7 to 15 mmol/g;
$b_2$) 0 to 50% by weight, more preferably 0 to 40% by weight, and most preferably 0 to 30% by weight of an organohydrogenpolysiloxane having at least two, preferably at least three SiH groups and a SiH content of 0.1

to 15 mmol/g, preferably from 4 to 15 mmol/g, more preferably from 7 to 15 mmol/g, and most preferably from 7 to 13 mmol/g, and a viscosity between 5 and 2,000 mPa·s, the total of the weight percentages of compounds $b_1$) and $b_2$) being at least 1% by weight;
c) 0.00001 to 0.2% by weight, preferably 0.0005 to 0.1% by weight, based on pure metal, of at least one catalyst to accelerate the hydrosilylation reaction;
d) 0 to 50% by weight, more preferably 0.1 to 40% by weight, and most preferably 0.5 to 35% by weight of reinforcing fillers having a BET surface area of at least 50 $m^2$/g;
e) 0 to 90% by weight, more preferably 0 to 80% by weight, and most preferably 0 to 75% y weight of non-reinforcing fillers having a BET surface area of less than 50 $m^2$/g and an average grain size of at least 0.1 μm;
f) 0 to 5% by weight, preferably 0 to 2% by weight of at least one dye;
g) 0 to 30% by weight, preferably 0 to 5% by weight of a moisture-binding agent;
h) 0 to 1% by weight, preferably 0 to 0.6% by weight, and most preferably 0 to 0.1% by weight of inhibitors;
i) 0 to 40% by weight, more preferably 0 to 30% by weight, and most preferably 0 to 20% by weight of at least one vinyl group-containing and/or SiH- containing and/or SiOR-containing (wherein R=H or alkyl) QM resin having a SiH content of 0 to 15 mmol/g, more preferably from 0 to 1 mmol/g and/or a SiOR content of 0 to 0.5 mmol/g;
j) 0 to 80% by weight, preferably 0 to 50% by weight of compounds of vinyl group-containing organopolysiloxanes and reinforcing fillers;
k) 0 to 10% by weight, preferably 0 to 5% by weight of surfactants, emulsifiers and/or stabilizers;
l) 0 to 90% by weight, preferably 0 to 80% by weight of radiopaque substances;
m) 0 to 20% by weight, preferably 0 to 10% by weight of $H_2$ absorbers/adsorbers or substances reducing or eliminating the generation of $H_2$; and
n) 0 to 20% by weight, preferably 0 to 15% by weight of additional auxiliary agents and additives.

**20.** The use of a dental material according to any one of claims 1 to 19 for producing bite registration material, cement, temporary crown and bridge material, temporary filler material and/or permanent filler material.

## Revendications

**1.** Matériau dentaire à réticulation par réaction d'addition à travers l'hydrosilylation, contenant

a) au moins un composé dont la molécule présente des groupements vinyle,
b) au moins un composé de type silicium organohydrogéné et
c) au moins un catalyseur,
**caractérisé en ce que** le matériau dentaire, à l'état complètement vulcanisé, présente une dureté Shore-D (selon DIN 53505) supérieure à 69 et/ou une résistance à la flexion d'au moins 8 MPa et/ou, dans essai de résistance à la flexion et mesuré selon ISO 10477, un module de Young d'au moins 300 MPa, et que l'au moins un composé a) et/ou l'au moins un composé b) comprend,
en tant que première unité structurelle, au moins un groupement volumineux et/ou rigide choisi dans le groupe constitué des groupements alkyle tertiaires, alkyle quartaires, cycloalkyle, cycloalcényle, aryle, aralkyle, alkylaryle, des groupements alkyle tertiaires pourvus de substituants halogène, des groupements alkyle quartaires pourvus de substituants halogène, des groupements aryle pourvus de substituants halogène, des groupements alkylaryle pourvus de substituants halogène, s'agissant avec une préférence particulière de groupes mono-, bis-, oligo- , polycycliques de nature aromatique ou non-aromatique, de groupements bisphénol A, bisphénol B, bisphénol F, de groupements 1,1,1-tris(4-hydroxyphényl)alcane, de groupements norbornane, de groupements adamantane et de groupements pentaérythritol, ainsi que,
en tant que deuxième unité structurelle, au moins deux unités silyle présentant des fonctions alcényle et/ou au moins au moins deux, de préférence au moins trois, unités silyle présentant des fonctions hydrogène, répondant à la formule générale I

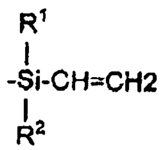 (Ia)

et/ou

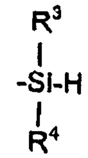 (Ib)

$R^1$, $R^2$ étant, indépendamment l'un de l'autre, choisis dans le groupe constitué par des groupements alkyle, des groupements alcényle, des groupements aryle, des groupements aralkyle, des groupements alkylaryle, des groupements alkyle halogénés, des groupements aryle halogénés, des groupements aralkyle halogénés, des groupements alkylaryle halogénés, des groupements cyanoalkyle, des groupement siloxy, des groupements cycloalkyle et des groupements cycloalcényle, s'agissant préférentiellement de groupements alkyle et, avec une préférence particulière, de groupements méthyle, et
$R^3$, $R^4$ étant, indépendamment l'un de l'autre, H ou $R^1$, s'agissant préférentiellement de groupements alkyle et, avec une préférence particulière, de groupements méthyle,
la deuxième unité structurelle étant reliée

i) directement,
ii) à travers un atome d'oxygène,
iii) à travers un groupement espaceur choisi dans le groupe $O-CH_2CH_2-$, $-O-(CH_2)_4-$, $-O-CR^1R^2-CR^1R^2-$, $CH_2CH_2CH_2(OCH_2CH_2)_4-$, $-CH_2-CH_2-CH_2-$, $-O-C\ (CH_3)_2-CH_2-$, $-O-CH(CH_3)-CH_2-$, $-O-CH(C_2H_5)-CH_2-$, $-O-CH(C_4H_9)-CH_2-$ , et $-O-CH-\ (C_{10}H_{21})\ -CH_2-$, $R^1$ et $R^2$ ayant indépendamment l'un de l'autre la signification définie ci-dessus, ou
iv) à travers un atome d'oxygène en combinaison avec un groupement espaceur selon iii),
à ladite première unité structurelle, l'au moins un composé dont la molécule présente des groupements vinyle a) ayant une teneur en Si-vinyle comprise entre 2 et 10 mmol/g et l'au moins un composé de type silicium organohydrogéné b) ayant une teneur en Si-H comprise entre 4 et 15 mmol/g.

2. Matériau dentaire selon la revendication 1,
**caractérisé en ce que** la distance entre les au moins deux groupements vinyle de l'au moins un composé a) et/ou la distance entre les au moins deux groupements SiH de l'au moins un composé b) est d'au moins 0,3 nm, préférentiellement d'au moins 0,45 nm, plus préférentiellement encore d'au moins 0,5 nm et, avec la plus grande préférence, d'au moins 0,7 nm.

3. Matériau dentaire selon les revendications 1 ou 2, **caractérisé en ce que** la première unité structurelle de l'au moins un composé a) et/ou de l'au moins un composé b) est un composé mono-, bis-, oligo- ou polycyclique aromatique et/ou un composé mono-, bis-, oligo- ou polycyclique non-aromatique, s'agissant avec une préférence particulière d'un groupement bisphénol A, bisphénol B, bisphénol F, d'un groupement 1,1,1- tris(4-hydroxyphényl) alcane, d'un groupement norbornane, d'un groupement adamantane ou d'un groupement pentaérythritol.

4. Matériau dentaire selon l'une des revendications précédentes, **caractérisé en ce que** la première unité structurelle de l'au moins un composé a) et/ou de l'au moins un composé b) est choisie dans le groupe constitué de

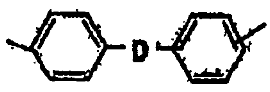 , 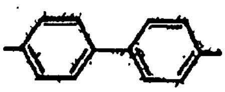 , 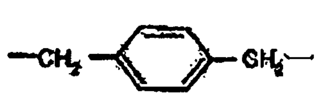

o, m, p 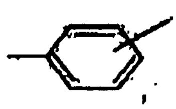 , 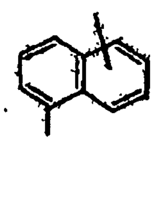 , 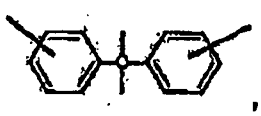 , 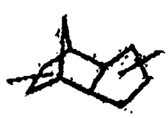

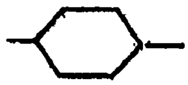 , 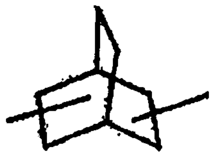 , 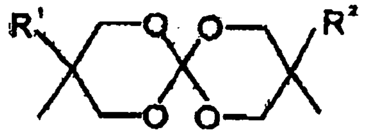

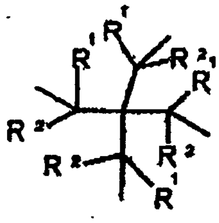 , 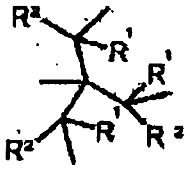 , 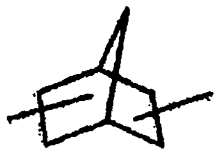

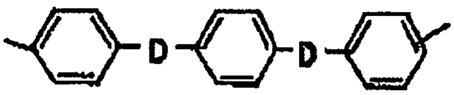 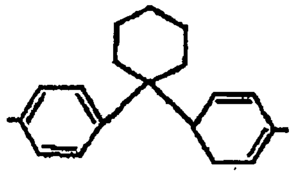

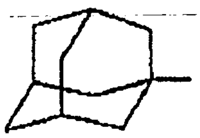 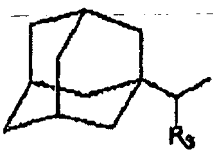 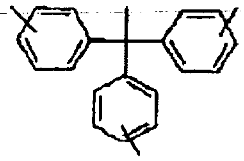

$R^1$, $R^2$, $R^3$ étant définis comme dans les formules I,
D = $CR^3R^4$, $R^3$ et $R^4$ étant de l'hydrogène ou répondant à la définition dans la formule Ib, D étant notamment égal à $CH_2$, $C(CH_3)_2$, CMePh avec Me = $CH_3$ et Ph = $C_6H_5$ ou à $CPh_2$

**5.** Matériau dentaire selon l'une des revendications précédentes, **caractérisé en ce que** la première unité structurelle de l'au moins un composé a) et/ou de l'au moins un composé b) est du bisphénol A, un dérivé de bisphénol A ou un composé de type trisphénol selon l'une des deux formules générales

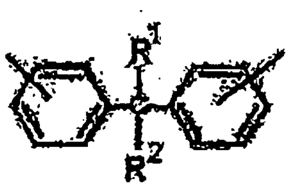 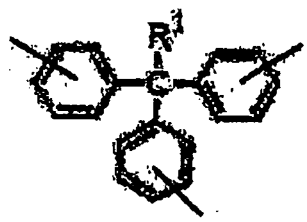

$R^1$, $R^2$ correspondant à la définition ci-dessus.

**6.** Matériau dentaire selon l'une des revendications précédentes, **caractérisé en ce que** l'au moins un composé a) et/ou l'au moins un composé b) est/contient une substance répondant aux formules générales II

$$H_2C{=}CH\text{-}SiR^1R^2\text{-}E_{n1}\text{-}SiR^1R^2\text{-}CH{=}CH_2 \qquad \text{(IIa)}$$

et/ou

$$H\text{-}SiR^3R^4\text{-}E_{n1}\text{-}SiR^3R^4\text{-}H \qquad \text{(IIb)},$$

les radicaux $R^1$, $R^2$, $R^3$, $R^4$ étant définis comme dans la formules I, E désignant la première unité structurelle et $n_1$ étant un nombre entier ≥ 1, de préférence 1.

7. Matériau dentaire selon l'une des revendications précédentes, **caractérisé en ce que** l'au moins un composé a) et/ou l'au moins un composé b) est/contient une substance répondant aux formules générales III

$$H_2C{=}CH\text{-}SiR^1R^2\text{-}O\text{-}E_{n1}\text{-}O\text{-}SiR^1R^2\text{-}CH{=}CH_2 \qquad \text{(IIIa)},$$

et/ou

$$H\text{-}SiR^3R^4\text{-}O\text{-}E_{n1}\text{-}O\text{-}SiR^3R^4\text{-}H \qquad \text{(IIIb)}$$

les radicaux $R^1$, $R^2$, $R^3$, $R^4$ et E ainsi que $n_1$ étant définis comme ci-dessus.

8. Matériau dentaire selon l'une des revendications précédentes, **caractérisé en ce que** l'au moins un composé a) et/ou l'au moins un composé b) est/contient une substance répondant aux formules générales IV

$$H_2C{=}CH\text{-}SiR^1R^2\text{-}A^1_{n2}\text{-}E_{n1}\text{-}A^2_{n3}\text{-}SiR^1R^2\text{-}CH{=}CH_2 \qquad \text{(IVa)}$$

et/ou

$$H\text{-}SiR^3R^4\text{-}A^1_{n2}\text{-}E_{n1}\text{-}A^2_{n3}\text{-}SiR^3R^4\text{-}H \qquad \text{(IVb)},$$

les radicaux $R^1$, $R^2$, $R^3$, $R^4$ et E ainsi que $n_1$ étant définis comme ci-dessus, $n_2$, $n_3$ étant identiques ou différents et, pour chacun d'entre eux, des nombres entiers ≥ 1, de préférence 1, $A^1$, $A^2$ étant, en outre et indépendamment l'un de l'autre, un espaceur selon la revendication 1.

9. Matériau dentaire selon l'une des revendications précédentes, **caractérisé en ce que** l'au moins un composé a) et/ou l'au moins un composé b) est constitué d'au moins une desdites premières unités structurelles ainsi que d'au moins une desdites deuxièmes unités structurelles, l'au moins une deuxième unité structurelle étant reliée à la première unité structurelle à travers un espaceur selon la revendication 1, lequel est lié à l'au moins une première unité structurelle à travers un atome d'oxygène.

10. Matériau dentaire selon l'une des revendications précédentes, **caractérisé en ce que** l'au moins un composé a) et/ou l'au moins un composé b) présente exactement un groupement rigide et/volumineux.

11. Matériau dentaire selon l'une des revendications précédentes, **caractérisé en ce qu'**il présente, à l'état complètement vulcanisé, une résistance à la flexion d'au moins 15 MPa qui, avec une préférence particulière, est d'au moins 19 MPa et/ou, dans l'essai de résistance à la flexion (mesuré selon ISO 10477), un module de Young d'au moins 600 MPa qui, avec une préférence particulière, est d'au moins 900 MPa.

12. Matériau dentaire selon l'une des revendications précédentes, **caractérisé en ce que** l'au moins un composé a) et l'au moins un composé b) contenant chacun une première et une deuxième unité structurelle selon la revendication 1.

13. Matériau dentaire selon l'une des revendications précédentes, **caractérisé en ce qu'**ils contiennent, outre l'au moins un composé a) et/ou l'au moins un composé b) présentant chacun une première et une deuxième unité structurelle selon la revendication 1, un ou plusieurs polysiloxanes organohydrogénés, s'agissant préférentiellement de ceux qui présentent au moins deux groupements Si-H par molécule et une teneur en Si-H comprise entre 4 et 14 et, avec une préférence particulière, entre 5 et 13 mmol/g, ainsi qu'une viscosité (à 20°C) comprise entre 5 et 2000 mPa.s.

14. Matériau dentaire selon l'une des revendications précédentes, **caractérisé en ce que** l'au moins un composé de

type silicium organohydrogéné b) est un composé répondant à la formule générale VI

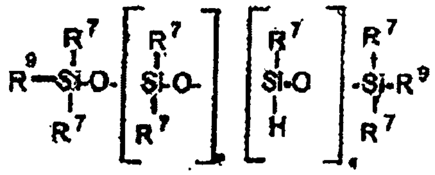

avec p = 0 à 1500
avec q = 0 à 1500, de préférence 2 à 1500
avec $R^7$ = groupements alkyle par exemple, méthyle, éthyle, isopropyle), aryle ( par exemple, phényle, naphtyle, tolyle, xylyle), aralkyle (benzyle, phényléthyle) ainsi que alkyle et aryle présentant des substituants halogènes (par exemple, 3,3,3-trifuoropropyle, chlorophényl, difluorophényl), cyanoalkyle. De préférence, R est égal à méthyle.
avec $R^9$ = $R^7$ et/ou H
à condition qu'au moins 2 atomes Si de la formule VI portent un atome H.

**15.** Matériau dentaire selon l'une des revendications précédentes, **caractérisé en ce que** l'au moins un composé de type silicium organohydrogéné b) est un composé répondant à la formule générale VII

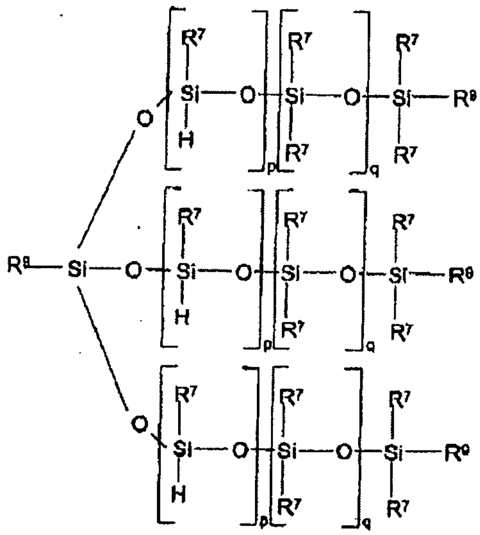

les radicaux ayant la signification indiquée dans la formule VI.

**16.** Matériau dentaire selon l'une des revendications précédentes, **caractérisé en ce que** l'au moins un composé de type silicium organohydrogéné b) est un composé répondant à la formule générale VIII

$$[SiO_{4/2}]\ [SiO_{1/3}R^1R^2H]_m$$

avec m = 0,9 à 4, de préférence m = 1 à 4.

**17.** Matériau dentaire selon l'une des revendications précédentes, **caractérisé en ce que** l'au moins un composé de type silicium organohydrogéné b) est une résine MQ contenant du Si-H qui est constituée d'unités $(R^9)_3SiO_{1/2}$, $(R^7)_2(H)SiO_{1/2}$ et $SiO_{4/2}$, le $(R^9)_1SiO_{3/2}$ trifonctionnel pouvant également être présent en tant qu'unités T et le $R^7R^9SiO_{2/2}$ bifonctionnel en tant qu'unités D, les radicaux $R^7$ et $R^9$ ayant les significations précédemment indiquées.

**18.** Matériau dentaire selon l'une des revendications précédentes, **caractérisé en ce que** l'au moins un composé de type organosilicium hydrogéné b), qui comprend une première et deuxième unité structurelle selon la revendication 1, présentant une teneur en SiH comprise entre 4 et 15 mmol/g et, avec une préférence particulière, entre 7 et 15 mmol/g ainsi qu'une viscosité (à 20°C) comprise entre 1 et 10000 mPa.s, de préférence entre 5 et 2000 mPa.s.

**19.** Matériau dentaire selon l'une des revendications précédentes, **caractérisé en ce qu'**il contient

$a_1$) 1 à 90 % en poids, de préférence 5 à 75 % en poids et, avec une préférence particulière, 10 à 60 % en poids, d'au moins un composé a) présentant au moins un groupe rigide et/ou volumineux ainsi qu'au moins deux groupements silyle dont la molécule présente des fonctions vinyle et une teneur en Si-vinyle comprise entre 2 et 10 mmol/g,
$a_2$) 0 à 40 % en poids, de préférence 0 à 30 % en poids et, avec une préférence particulière, 0 à 20 % en poids, d'au moins un organo-polysiloxane dont la molécule présente au moins deux groupements vinyle et une teneur en Si-vinyle comprise entre 0,5 et 10 mmol/g, la teneur en Si-vinyle étant préférentiellement comprise entre 1 et 10 mmol/g et, avec une préférence particulière, entre 2 et 10 mmol/g, ainsi qu'une viscosité comprise entre 21 et 350.000 mPa.s,
$b_1$) 0 à 90 % en poids, de préférence 5 à 75 % en poids et, avec une préférence particulière, 10 à 60 % en poids, d'un composé de type silicium organohydrogéné présentant au moins un groupement rigide et/ou volumineux ainsi qu'au moins deux groupements silyle pourvus d'au moins deux, préférentiellement de 3 groupements SiH et présentant une teneur en SiH comprise entre 4 et 15 mmol/g et, avec une préférence particulière, entre 7 et 15 mmol/g,
$b_2$) 0 à 50 % en poids, de préférence 0 à 40 % en poids et, avec une préférence particulière, 0 à 30 % en poids, d'un polysiloxane organohydrogéné présentant au moins deux, préférentiellement 3 groupement SiH et une teneur en SiH comprise entre 0,1 et 15 mmol/g, préférentiellement entre 4 et 15 mmol/g, avec une préférence particulière entre 7 et 15 mmol/g et, avec une préférence toute particulière, entre 7 et 13 mmol/g ainsi qu'une viscosité comprise entre 5 et 2000 mPa.s, la somme des pourcentages en poids des composés $b_1$) et $b_2$) étant d'au moins 1 % en poids,
c) 0,00001 à 0,2 % en poids, de préférence 0,0005 à 0,1 % en poids, d'au moins un catalyseur servant à accélérer la réaction d'hydrosilylation, par rapport à du métal pur,
d) 0 à 50 % en poids, préférentiellement 0,1 à 40 % en poids et, avec une préférence particulière, 0,5 à 35 % en poids de charges de renforcement ayant une surface BET d'au moins 50 $m^2$/g,
e) 0 à 90 % en poids, préférentiellement 0 à 80 % en poids et, avec une préférence particulière, 0 à 75 % en poids de charges non renfonçantes ayant une surface BET inférieure à 50 $m^2$/g une granulométrie moyenne d'au moins 0,1 $\mu$m,
f) 0 à 5 % en poids, de préférence 0 à 2 % en poids, d'au moins un colorant,
g) 0 à 30 % en poids, de préférence 0 à 5 % en poids, d'un agent de rétention d'humidité,
h) 0 à 1 % en poids, de préférence 0 à 0,6 et, avec une préférence particulière, 0 à 0,1 % en poids d'inhibiteurs,
i) 0 à 40 % en poids, préférentiellement 0 à 30 % en poids et, avec une préférence particulière 0 à 20 % en poids, d'au moins une résine QM contenant des groupements vinyle et/ou du SiH et/ou du SiOR (avec R = H ou alkyle) et présentant une teneur en SiH comprise entre 0 et 15 mmol/g, préférentiellement entre 0 et 1 mmol/g et une teneur en SiOR comprise entre 0 et 0,5 mmol/g.
j) 0 à 80 % en poids, de préférence 0 à 50 % en poids, de compounds constitués d'organopolysiloxanes contenant des groupements vinyle et de charges de renforcement,
k) 0 à 10 % en poids, de préférence 0 à 5 % en poids, d'agents tensioactifs, d'émulsifiants et/ou d'agents stabilisants,
l) 0 à 90 % en poids, de préférence 0 à 80 % en poids, de substances radio-opaques,
m) 0 à 20 % en poids, de préférence 0 à 10 % en poids, d'agent d'absorption/adsorption de $H_2$ ou de substances diminuant ou empêchant le développement de $H_2$ ainsi que
n) 0 à 20 % en poids, de préférence 0 à 15 % en poids, d'agents auxiliaires et d'additifs supplémentaires.

**20.** Utilisation d'un matériau dentaire selon l'une des revendications 1 à 19 pour préparer du matériau de prise d'empreinte dentaire, du ciment, du matériau provisoire pour couronnes et bridges et/ou du matériau destiné à réaliser des obturations permanentes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0522341 A1
- EP 0894117 B1

### In der Beschreibung aufgeführte Nicht-Patentliteratur

- UllmannEncyclopädie der Technischen Chemie. vol. 21, 523